# EUROPEAN PATENT APPLICATION

(11) **EP 1 486 566 A1**
(43) Date of publication of application: **15.12.2004**
(21) Application number: 03707172.7
(22) Date of filing: 28.02.2003
(51) Int. Cl.: C12N 15/13, C12N 1/15, C12N 1/19, C12N 1/21, C12N 5/10, C07K 16/44, C12P 21/02, G01N 33/53

(54) **PROTEINS CAPABLE OF BINDING TO FEMALE SEX HORMONES AND PROCESS FOR PRODUCING THE SAME**

(30) Priority: 01.03.2002 JP 2002055669
(71) Applicant: Japan Envirochemicals, Ltd., Osaka-shi, Osaka 541-0045 (JP)
(72) Inventor: KATAGIRI, Masanao, Osaka-shi, Osaka 532-0026 (JP); FUJIMOTO, Shigeru Japan EnviroChemicals, Ltd., Osaka-shi, Osaka (JP); GODA, Yasuhiro, Japan EnviroChemicals, Ltd., Osaka-shi, Osaka (JP)
(74) Representative: Helbing, Jörg, Dr. Dipl.-Chem.
(86) International application number: PCT/JP2003/002311
(87) International publication number: WO 2003/074704

(57) **Abstract**

The present invention provides a protein capable of binding to a female sex hormone, which protein is supplemented with useful properties for measuring, quantifying and concentrating female sex hormones, such as high sensitivity, low cross-reactivity, unlikelihood of being influenced by interfering substances, and unlikelihood of being influenced by solvents. Specifically, the present invention provides a recombinant protein prepared by obtaining various genes for various antibodies against female sex hormones, and modifying, by gene recombination technology, various properties of the original antibody, such as affinity, avidity, cross-reactivity, resistance for interfering substance on antigen-antibody reaction, resistance for interfering substance on enzymatic color developing reaction, resistance for solvent and the like.

## Description

### Technical Field

The present invention relates to an anti-female sex hormone antibody, a gene encoding the same, a protein capable of binding to said female sex hormones and a process of producing the same, a method of determining or quantifying female sex hormones, a method of concentrating female sex hormones and the like.

### Background Art

Environmental pollution due to the female sex hormones has been a problem in recent years. It is necessary to measure and analyze female sex hormones and degradation products thereof in the environment, and make use of the results for the preservation of the environment.

As such measurement and analysis method, the method described in JP-A-2001-41958 is a superior method.

### Disclosure of Invention

The present invention aims at preparing and utilizing a protein capable of binding to female sex hormones, which is obtained by supplementing a recombinant protein prepared by obtaining various genes for antibodies against female sex hormones, and modifying, by gene recombination technology, various properties of the original antibody, such as affinity, avidity, cross-reactivity, resistance for interfering substance on antigen-antibody reaction, resistance for interfering substance on enzymatic color developing reaction, resistance for solvent and the like, with useful properties for measuring, quantifying or concentrating female sex hormones, such as high sensitivity, low cross-reactivity, unlikelihood of being influenced by interfering substances, unlikelihood of being influenced by solvents and the like.

As the female sex hormones, for example, estrogens such as estradiol (E2), estrone (E1), estriol (E3) and the like, synthetic female sex hormones such as ethynyl estradiol (EE2), diethylbestrol (DES) and the like, and the like can be mentioned.

The present inventors have conducted intensive studies to obtain a protein capable of binding to female sex hormones, which is supplemented with useful properties, such as capability of high sensitive measurement and the like, by improving affinity, and found that the protein capable of binding to female sex hormones can be efficiently produced by preparing a transformant retaining its gene or an engineered gene, and further studied to complete the present invention.

Accordingly, the present invention provides
(1) a protein of the following [a] or [b], or a salt thereof:
   [a] a protein containing a heavy chain variable region of an anti-female sex hormone antibody, which has an amino acid sequence shown by SEQ ID No: 2, an amino acid sequence shown by SEQ ID No: 6, an amino acid sequence shown by SEQ ID No: 10, an amino acid sequence shown by SEQ ID No: 14, an amino acid sequence shown by SEQ ID No: 18 or an amino acid sequence substantially the same as these;
   [b] a protein containing a light chain variable region of an anti-female sex hormone antibody, which has an amino acid sequence shown by SEQ ID No: 4, an amino acid sequence shown by SEQ ID No: 8, an amino acid sequence shown by SEQ ID No: 12, an amino acid sequence shown by SEQ ID No: 16, an amino acid sequence shown by SEQ ID No: 20 or an amino acid sequence substantially the same as these;
(2) a protein of any of the following [a¹]-[a³] and [b¹]-[b³] or a salt thereof:
   [a¹] a protein consisting of an amino acid sequence of any of the following (i)-(v), wherein a particular region is selected from the group consisting of a complementarity determining region 1, a complementarity determining region 2, a complementarity determining region 3, a framework region 1, a framework region 2, a framework region 3 and a framework region 4,
      (i) an amino acid sequence wherein an amino acid sequence corresponding to not less than one particular region of the amino acid sequence shown by SEQ ID No: 2 has been exchanged for an amino acid sequence corresponding to the same kind of not less than one particular region contained in an amino acid sequence shown by SEQ ID No: 6, an amino acid sequence shown by SEQ ID No: 10, an amino acid sequence shown by SEQ ID No: 14 or an amino acid sequence shown by SEQ ID No: 18,
      (ii) an amino acid sequence wherein an amino acid sequence corresponding to not less than one particular region of the amino acid sequence shown by SEQ ID No: 6 has been exchanged for an amino acid sequence corresponding to the same kind of not less than one particular region contained in an amino acid sequence shown by SEQ ID No: 2, an amino acid sequence shown by SEQ ID No: 10, an amino acid sequence shown by SEQ ID No: 14 or an amino acid sequence shown by SEQ ID No: 18,
      (iii) an amino acid sequence wherein an amino acid sequence corresponding to not less than one particular region of the amino acid sequence shown by SEQ ID No: 10 has been exchanged for an amino acid sequence corresponding to the same kind of not less than one particular region contained in an amino acid sequence shown by SEQ ID No: 2, an amino acid sequence shown by SEQ ID No: 6, an amino acid sequence shown by SEQ ID No: 14 or an amino acid sequence shown by SEQ ID No: 18,
      (iv) an amino acid sequence wherein an amino acid sequence corresponding to not less than one particular region of the amino acid sequence shown by SEQ ID No: 14 has been exchanged for an amino acid sequence corresponding to the same kind of not less than one particular region contained in an amino acid sequence shown by SEQ ID No: 2, an amino acid sequence shown by SEQ ID No: 6, an amino acid sequence shown by SEQ ID No: 10 or an amino acid sequence shown by SEQ ID No: 18, and
      (v) an amino acid sequence wherein an amino acid sequence corresponding to not less than one particular region of the amino acid sequence shown by SEQ ID No: 18 has been exchanged for an amino acid sequence corresponding to the same kind of not less than one particular region contained in an amino acid sequence shown by SEQ ID No: 2, an amino acid sequence shown by SEQ ID No: 6, an amino acid sequence shown by SEQ ID No: 10 or an amino acid sequence shown by SEQ ID No: 14;
   [b¹] a protein consisting of an amino acid sequence of any of the following (i) - (v), wherein a particular region is selected from the group consisting of a complementarity determining region 1, a complementarity determining region 2, a complementarity determining region 3, a framework region 1, a framework region 2, a framework region 3 and a framework region 4,
      (i) an amino acid sequence wherein an amino acid sequence corresponding to not less than one particular region of the amino acid sequence shown by SEQ ID No: 4 has been exchanged for an amino acid sequence corresponding to the same kind of not less than one particular region contained in an amino acid sequence shown by SEQ ID No: 8, an amino acid sequence shown by SEQ ID No: 12, an amino acid sequence shown by SEQ ID No: 16 or an amino acid sequence shown by SEQ ID No: 20,
      (ii) an amino acid sequence wherein an amino acid sequence corresponding to not less than one particular region of the amino acid sequence shown by SEQ ID No: 8 has been exchanged for an amino acid sequence corresponding to the same kind of not less than one particular region contained in an amino acid sequence shown by SEQ ID No: 4, an amino acid sequence shown by SEQ ID No: 12, an amino acid sequence shown by SEQ ID No: 16 or an amino acid sequence shown by SEQ ID No: 20,
      (iii) an amino acid sequence wherein an amino acid sequence corresponding to not less than one particular region of the amino acid sequence shown by SEQ ID No: 12 has been exchanged for an amino acid sequence corresponding to the same kind of not less than one particular region contained in an amino acid sequence shown by SEQ ID No: 4, an amino acid sequence shown by SEQ ID No: 8, an amino acid sequence shown by SEQ ID No: 16 or an amino acid sequence shown by SEQ ID No: 20,
      (iv) an amino acid sequence wherein an amino acid sequence corresponding to not less than one particular region of the amino acid sequence shown by SEQ ID No: 16 has been exchanged for an amino acid sequence corresponding to the same kind of not less than one particular region contained in an amino acid sequence shown by SEQ ID No: 4, an amino acid sequence shown by SEQ ID No: 8, an amino acid sequence shown by SEQ ID No: 12 or an amino acid sequence shown by SEQ ID No: 20, and
      (v) an amino acid sequence wherein an amino acid sequence corresponding to not less than one particular region of the amino acid sequence shown by SEQ ID No: 20 has been exchanged for an amino acid sequence corresponding to the same kind of not less than one particular region contained in an amino acid sequence shown by SEQ ID No: 4, an amino acid sequence shown by SEQ ID No: 8, an amino acid sequence shown by SEQ ID No: 12 or an amino acid sequence shown by SEQ ID No: 16;
   [a²] a protein having an amino acid sequence shown by SEQ ID No: 2, an amino acid sequence shown by SEQ ID No: 6, an amino acid sequence shown by SEQ ID No: 10, an amino acid sequence shown by SEQ ID No: 14, an amino acid sequence shown by SEQ ID No: 18 or an amino acid sequence of the protein of [a¹], wherein one or more amino acids have been deleted, substituted or added, which binds to a female sex hormone after forming a complex with a protein having an amino acid sequence shown by SEQ ID No: 4, an amino acid sequence shown by SEQ ID No: 8, an amino acid sequence shown by SEQ ID No: 12, an amino acid sequence shown by SEQ ID No: 16, an amino acid sequence shown by SEQ ID No: 20 or an amino acid sequence of the protein of [b¹];
   [b²] a protein having an amino acid sequence shown by SEQ ID No: 4, an amino acid sequence shown by SEQ ID No: 8, an amino acid sequence shown by SEQ ID No: 12, an amino acid sequence shown by SEQ ID No: 16, an amino acid sequence shown by SEQ ID No: 20 or an amino acid sequence of the protein of [b¹], wherein one or more amino acids have been deleted, substituted or added, which binds to a female sex hormone after forming a complex with a protein having an amino acid sequence shown by SEQ ID No: 2, an amino acid sequence shown by SEQ ID No: 6, an amino acid sequence shown by SEQ ID No: 10, an amino acid sequence shown by SEQ ID No: 14, an amino acid sequence shown by SEQ ID No: 18 or an amino acid sequence of the protein of [a¹];
   [a³] a protein having an amino acid sequence shown by SEQ ID No: 2, an amino acid sequence shown by SEQ ID No: 6, an amino acid sequence shown by SEQ ID No: 10, an amino acid sequence shown by SEQ ID No: 14, an amino acid sequence shown by SEQ ID No: 18 or an amino acid sequence of the protein of [a¹], wherein one or more amino acids have been deleted, substituted or added, which binds to a female sex hormone after forming a complex with a protein having an amino acid sequence shown by SEQ ID No: 4, an amino acid sequence shown by SEQ ID No: 8, an amino acid sequence shown by SEQ ID No: 12, an amino acid sequence shown by SEQ ID No: 16, an amino acid sequence shown by SEQ ID No: 20 or an amino acid sequence of the protein of [b¹], wherein one or more amino acids have been deleted, substituted or added;
   [b³] a protein having an amino acid sequence shown by SEQ ID No: 4, an amino acid sequence shown by SEQ ID No: 8, an amino acid sequence shown by SEQ ID No: 12, an amino acid sequence shown by SEQ ID No: 16, an amino acid sequence shown by SEQ ID No: 20 or an amino acid sequence of the protein of [b¹], wherein one or more amino acids have been deleted, substituted or added, which binds to a female sex hormone after forming a complex with a protein having an amino acid sequence shown by SEQ ID No: 2, an amino acid sequence shown by SEQ ID No: 6, an amino acid sequence shown by SEQ ID No: 10, an amino acid sequence shown by SEQ ID No: 14, an amino acid sequence shown by SEQ ID No: 18 or an amino acid sequence of the protein of [a¹], wherein one or more amino acids have been deleted, substituted or added;
(3) the protein of the above-mentioned (1) or (2), or a salt thereof, wherein the female sex hormone is estrogen or a synthetic female sex hormone;
(4) the protein of the above-mentioned (3), or a salt thereof, wherein the estrogen is estradiol, estrone or estriol;
(5) the protein of the above-mentioned (3), or a salt thereof, wherein the synthetic female sex hormone is ethynyl estradiol or diethylbestrol;
(6) a method of genetically recombining a protein of any of the above-mentioned (1)-(5);
(7) a protein obtained by the method of the above-mentioned (6), or a salt thereof;
(8) a partial peptide of a protein of any of the above-mentioned (1)-(5) and (7), or a salt thereof;
(9) a polynucleotide encoding a protein or partial peptide of a protein of any of the above-mentioned (1)-(5) and (7);
(10) a recombinant vector containing a polynucleotide of the above-mentioned (9);
(11) a transformant transformed with the recombinant vector of the above-mentioned (10);
(12) a method of producing a protein of any of the above-mentioned (1)-(5) and (7), or a partial peptide thereof, or a salt thereof, which comprises producing a protein of any of the above-mentioned (1)-(5) and (7), or a partial peptide thereof, or a salt thereof, and harvesting the same;
(13) a complex wherein a protein of the following [a] and a protein of the following [b] are linked:
   [a] a protein having an amino acid sequence shown by SEQ ID No: 2, an amino acid sequence shown by SEQ ID No: 6, an amino acid sequence shown by SEQ ID No: 10, an amino acid sequence shown by SEQ ID No: 14, an amino acid sequence shown by SEQ ID No: 18 or an amino acid sequence substantially the same as these;
   [b] a protein having an amino acid sequence shown by SEQ ID No: 4, an amino acid sequence shown by SEQ ID No: 8, an amino acid sequence shown by SEQ ID No: 12, an amino acid sequence shown by SEQ ID No: 16, an amino acid sequence shown by SEQ ID No: 20 or an amino acid sequence substantially the same as these;
(14) a complex of the above-mentioned (13), which is a single chain antibody;
(15) a phage that expresses the single chain antibody of the above-mentioned (14);
(16) a method of identifying a female sex hormone binding to a complex of the above-mentioned (13) or (14), which comprises using said complex or a protein of any of the above-mentioned (1)-(5) and (7) or the phage of the above-mentioned (15);
(17) a method of measuring or quantifying a female sex hormone, which comprises using the complex of the above-mentioned (13) or (14) or a protein of any of the above-mentioned (1)-(5) and (7) or the phage of the above-mentioned (15);
(18) a kit for measuring or quantifying a female sex hormone, which comprises the complex of the above-mentioned (13) or (14) or a protein of any of the above-mentioned (1)-(5) and (7) or the phage of the above-mentioned (15);
(19) a method of concentrating a female sex hormone, which comprises using the complex of the above-mentioned (13) or (14) or a protein of any of the above-mentioned (1)-(5) and (7) or the phage of the above-mentioned (15);
(20) a kit for concentrating a female sex hormone, which comprises the complex of the above-mentioned (13) or (14) or a protein of any of the above-mentioned (1)-(5) and (7) or the phage of the above-mentioned (15);
(21) a mouse hybridoma cell, which is E2-73 strain (FERM BP-7569), E1-420 strain (FERM BP-7568), EE2-227 strain (FERM BP-7567), EE2-3 strain or EE2-8-18 strain;
(22) a monoclonal antibody produced by the mouse hybridoma cell of the above-mentioned (21); and the like.

### Brief Description of Drawings

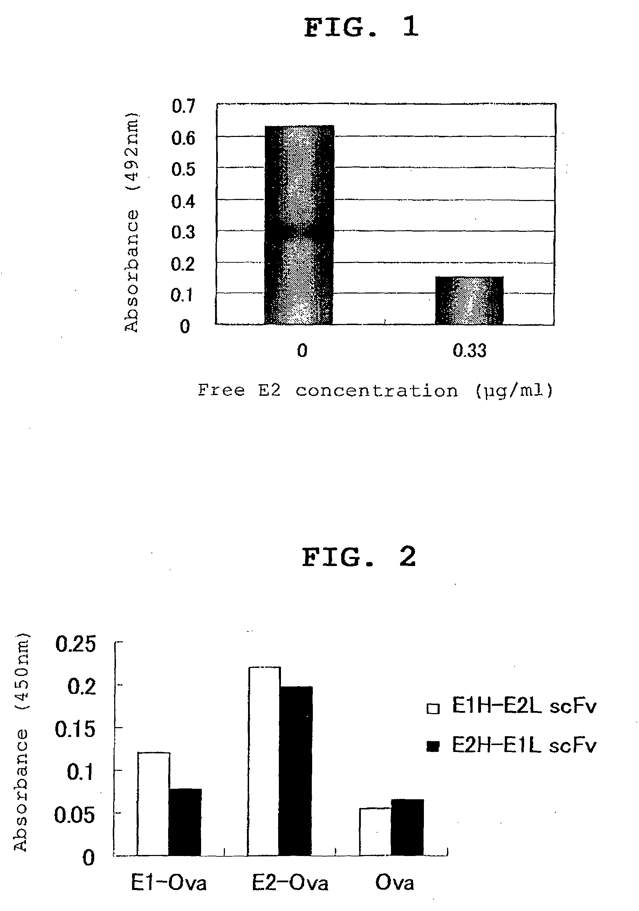

Fig. 1 confirms that an anti-estradiol single chain variable region antibody constructed in the present invention binds to 17β-estradiol.
Fig. 2 confirms that an anti-single chain variable region antibody (E1H-E2L scFv, E2H-E1L scFv) constructed in the present invention binds to estrone and 17β-estradiol, wherein white columns correspond to E1H-E2L scFv and black columns correspond to E2H-E1L scFv, respectively. E1-Ova means that a complex of estrone and ovalbumin is an antigen, E2-Ova means that a complex of 17β-estradiol and ovalbumin is an antigen, and Ova means that ovalbumin is an antigen.

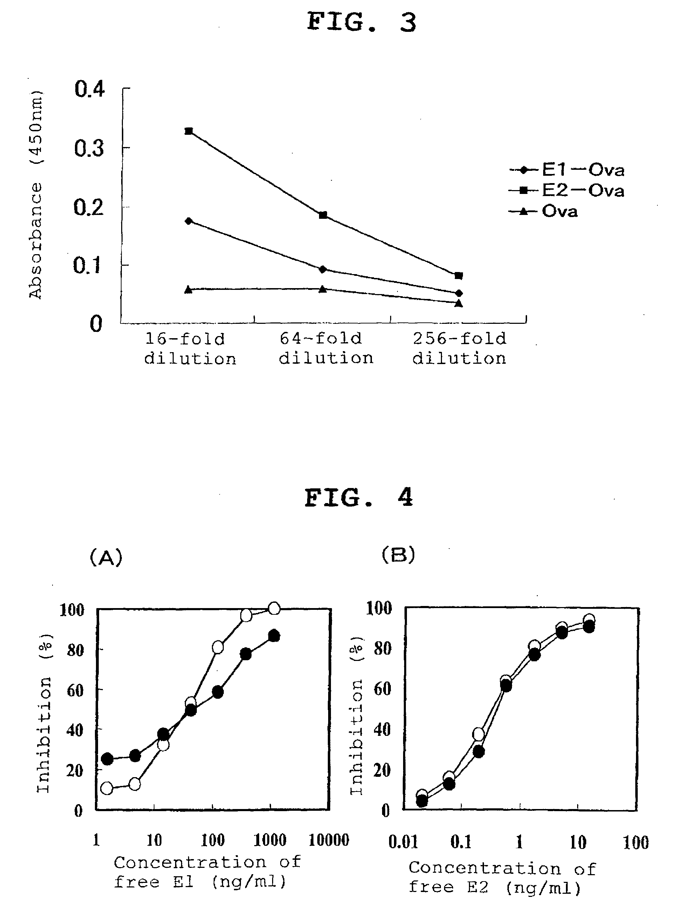

Fig. 3 confirms changes in the binding ability to estrone and 17β-estradiol, due to dilution of a phage that expresses an anti-single chain variable region antibody (E2H-E1L scFv) constructed in the present invention. E1-Ova means that a complex of estrone and ovalbumin is an antigen, and E2-Ova means that a complex of 17β-estradiol and ovalbumin is an antigen, and Ova means that ovalbumin is an antigen.
Fig. 4 confirms antigen binding ability of an anti-single chain variable region antibody constructed in the present invention and the original monoclonal antibody. Fig. 4(A) shows estrone binding ability of an anti-estrone single chain variable region antibody and monoclonal antibody E1-420. Fig. 4(B) shows 17β-estradiol binding ability of an anti-estradiol single chain variable region antibody and anti-estradiol monoclonal antibody E2-733. The vertical axis (% inhibition) of Figs. 4(A) and 4(B) shows the degree of inhibition of the binding of an antibody to an antigen on a plate by a free antigen, as expressed by subtracting absorbance at each concentration (%) from absorbance without free antigen, which is taken as 100%.

### Detailed Description of the Invention

The present invention provides [a] a protein having (or consisting of) an amino acid sequence shown by SEQ ID No: 2, an amino acid sequence shown by SEQ ID No: 6, an amino acid sequence shown by SEQ ID No: 10, an amino acid sequence shown by SEQ ID No: 14, an amino acid sequence shown by SEQ ID No: 18 or an amino acid sequence substantially the same as these; or [b] a protein having (or consisting of) an amino acid sequence shown by SEQ ID No: 4, an amino acid sequence shown by SEQ ID No: 8, an amino acid sequence shown by SEQ ID No: 12, an amino acid sequence shown by SEQ ID No: 16, an amino acid sequence shown by SEQ ID No: 20 or an amino acid sequence substantially the same as these. The above-mentioned protein [a] is a protein having an amino acid sequence of a heavy chain variable region of an anti-female sex hormone antibody or an amino acid sequence substantially the same as said amino acid sequence. On the other hand, the above-mentioned protein [b] is a protein having an amino acid sequence of a light chain variable region of an anti-female sex hormone antibody or an amino acid sequence substantially the same as said amino acid sequence. These are sequentially explained in the following.

The above-mentioned protein [a] is not particularly limited as long as it has an amino acid sequence of a heavy chain variable region of an anti-female sex hormone antibody or an amino acid sequence substantially the same as said amino acid sequence. In one embodiment, the above-mentioned protein [a] can be a protein of the above-mentioned [a¹], [a²] or [a³] . In addition, the protein of the above-mentioned [a¹] is not particularly limited as long as it is a protein consisting of an amino acid sequence wherein particular regions of the amino acid sequence shown by SEQ ID No: 2, the amino acid sequence shown by SEQ ID No: 6, an amino acid sequence shown by SEQ ID No: 10, an amino acid sequence shown by SEQ ID No: 14 or an amino acid sequence shown by SEQ ID No: 18 are exchanged for particular regions of a different amino acid sequence, and a protein consisting of any of the above-mentioned (i) - (v) can be specifically mentioned.

As the particular region in the above-mentioned [a¹], complementarity determining region 1, complementarity determining region 2, complementarity determining region 3 (hereinafter to be abbreviated as CDR1, CDR2, CDR3 as necessary), framework region 1, framework region 2, framework region 3, framework region 4 (hereinafter to be abbreviated as FR1, FR2, FR3, FR4 as necessary) can be mentioned. In the above-mentioned [a¹], the amino acid sequence to be the object of exchange is preferably an amino acid sequence of the same kinds of particular regions. Therefore, for example, a protein having an amino acid sequence corresponding to CDR1 in an amino acid sequence shown by SEQ ID No: 6, and an amino acid sequence corresponding to CDR2, CDR3, FR1, FR2, FR3, FR4 in an amino acid sequence shown by SEQ ID No: 2 is provided. In addition, while the number of the particular regions to be exchanged is not particularly limited as long as it is not less than 1, it is, for example, 1 to 3, preferably 1 or 2, more preferably 1. The amino acid sequences can be exchanged by a method known *per se.* Specifically, a primer wherein a part corresponding to the regions to be exchanged is linked to a primer corresponding to both the N, C terminals of each region is designed, and using this primer, a fragment is amplified by PCR, and a PCR is performed with the exchanged combination.

In an amino acid sequence shown by SEQ ID No: 2, SEQ ID No: 6, SEQ ID No: 10, SEQ ID No: 14 or SEQ ID No: 18, the regions corresponding to CDR1, CDR2, CDR3, FR1, FR2, FR3, FR4 are specifically as follows:
(i) CDR1 (25th to 34th amino acid residues of an amino acid sequence shown by SEQ ID No: 2, 25th to 34th amino acid residues of an amino acid sequence shown by SEQ ID No: 6, 25th to 36th amino acid residues of an amino acid sequence shown by SEQ ID No: 10, 25th to 34th amino acid residues of an amino acid sequence shown by SEQ ID No: 14 and 25th to 36th amino acid residues of an amino acid sequence shown by SEQ ID No: 18);
(ii) CDR2 (49th to 58th amino acid residues of an amino acid sequence shown by SEQ ID No: 2, 49th to 59th amino acid residues of an amino acid sequence shown by SEQ ID No: 6, 51st to 59th amino acid residues of an amino acid sequence shown by SEQ ID No: 10, 49th to 57th amino acid residues of an amino acid sequence shown by SEQ ID No: 14 and 51st to 59th amino acid residues of an amino acid sequence shown by SEQ ID No: 18);
(iii) CDR3 (96th to 106th amino acid residues of an amino acid sequence shown by SEQ ID No: 2, 98th to 106th amino acid residues of an amino acid sequence shown by SEQ ID No: 6, 99th to 107th amino acid residues of an amino acid sequence shown by SEQ ID No: 10, 97th to 107th amino acid residues of an amino acid sequence shown by SEQ ID No: 14 and 99th to 107th amino acid residues of an amino acid sequence shown by SEQ ID No: 18);
(iv) FR1 (1st to 24th amino acid residues of an amino acid sequence shown by SEQ ID No: 2, 1st to 24th amino acid residues of an amino acid sequence shown by SEQ ID No: 6, 1st to 24th amino acid residues of an amino acid sequence shown by SEQ ID No: 10, 1st to 24th amino acid residues of an amino acid sequence shown by SEQ ID No: 14 and 1st to 24th amino acid residues of an amino acid sequence shown by SEQ ID No: 18);
(v) FR2 (35th to 48th amino acid residues of an amino acid sequence shown by SEQ ID No: 2, 35th to 48th amino acid residues of an amino acid sequence shown by SEQ ID No: 6, 37th to 50th amino acid residues of an amino acid sequence shown by SEQ ID No: 10, 35th to 48th amino acid residues of an amino acid sequence shown by SEQ ID No: 14 and 37th to 50th amino acid residues of an amino acid sequence shown by SEQ ID No: 18);
(vi) FR3 (59th to 95th amino acid residues of an amino acid sequence shown by SEQ ID No: 2, 60th to 97th amino acid residues of an amino acid sequence shown by SEQ ID No: 6, 60th to 98th amino acid residues of an amino acid sequence shown by SEQ ID No: 10, 58th to 96th amino acid residues of an amino acid sequence shown by SEQ ID No: 14 and 60th to 98th amino acid residues of an amino acid sequence shown by SEQ ID No: 18);
(vii) FR4 (107th to 117th amino acid residues of an amino acid sequence shown by SEQ ID No: 2, 107th to 118th amino acid residues of an amino acid sequence shown by SEQ ID No: 6, 108th to 118th amino acid residues of an amino acid sequence shown by SEQ ID No: 10, 108th to 118th amino acid residues of an amino acid sequence shown by SEQ ID No: 14 and 108th to 118th amino acid residues of an amino acid sequence shown by SEQ ID No: 18);

In another embodiment, the above-mentioned protein [a] is, for example, [a⁴] a protein having an amino acid sequence having significant homology to an amino acid sequence shown by SEQ ID No: 2, SEQ ID No: 6, SEQ ID No: 10, SEQ ID No: 14, SEQ ID No: 18 or an amino acid sequence of the protein of [a¹] above, which binds to a female sex hormone upon formation of a complex with a protein having an amino acid sequence having significant homology to an amino acid sequence shown by SEQ ID No: 4, SEQ ID No: 8, SEQ ID No: 12, SEQ ID No: 16, SEQ ID No: 20 or an amino acid sequence of the protein of [b] above.

The above-mentioned protein [b] is not particularly limited as long as it has an amino acid sequence of a light chain variable region of an anti-female sex hormone antibody or an amino acid sequence substantially the same as said amino acid sequence. In one embodiment, the above-mentioned protein [b] can be a protein of the above-mentioned [b¹], [b²] or [b³]. In addition, the protein of the above-mentioned [b¹] is not particularly limited as long as it is a protein consisting of an amino acid sequence wherein particular regions of the amino acid sequence shown by SEQ ID No: 4 or the amino acid sequence shown by SEQ ID No: 8, an amino acid sequence shown by SEQ ID No: 12, an amino acid sequence shown by SEQ ID No: 16 or an amino acid sequence shown by SEQ ID No: 20 are exchanged for particular regions of a different amino acid sequence, and a protein consisting of any of the above-mentioned (i) - (v) can be specifically mentioned.

As the particular regions in the above-mentioned [b¹], CDR1, CDR2, CDR3, FR1, FR2, FR3 and FR4 can be mentioned. In the above-mentioned [b¹], the amino acid sequence to be the object of exchange is preferably an amino acid sequence of the same kinds of particular regions. Therefore, for example, a protein having an amino acid sequence corresponding to CDR1 in an amino acid sequence shown by SEQ ID No: 4, and an amino acid sequence corresponding to CDR2, CDR3, FR1, FR2, FR3, FR4 in an amino acid sequence shown by SEQ ID No: 8 is provided. In addition, while the number of the particular regions to be exchanged is not particularly limited as long as it is not less than 1, it is, for example, 1 to 3, preferably 1 or 2, more preferably 1.

In an amino acid sequence shown by SEQ ID No: 4, SEQ ID No: 8, SEQ ID No: 12, SEQ ID No: 16 or SEQ ID No: 20, the regions corresponding to CDR1, CDR2, CDR3, FR1, FR2, FR3, FR4 are specifically as follows:
(i) CDR1 (24th to 34th amino acid residues of an amino acid sequence shown by SEQ ID No: 4, 24th to 39th amino acid residues of an amino acid sequence shown by SEQ ID No: 8, 24th to 40th amino acid residues of an amino acid sequence shown by SEQ ID No: 12, 24th to 34th amino acid residues of an amino acid sequence shown by SEQ ID No: 16 and 24th to 40th amino acid residues of an amino acid sequence shown by SEQ ID No: 20);
(ii) CDR2 (50th to 56th amino acid residues of an amino acid sequence shown by SEQ ID No: 4, 55th to 60th amino acid residues of an amino acid sequence shown by SEQ ID No: 8, 56th to 62nd amino acid residues of an amino acid sequence shown by SEQ ID No: 12, 50th to 56th amino acid residues of an amino acid sequence shown by SEQ ID No: 16, 56th to 62nd amino acid residues of an amino acid sequence shown by SEQ ID No: 20);
(iii) CDR3 (89th to 97th amino acid residues of an amino acid sequence shown by SEQ ID No: 4, 94th to 102nd amino acid residues of an amino acid sequence shown by SEQ ID No: 8, 95th to 102nd amino acid residues of an amino acid sequence shown by SEQ ID No: 12, 89th to 97th amino acid residues of an amino acid sequence shown by SEQ ID No: 16, 95th to 102nd amino acid residues of an amino acid sequence shown by SEQ ID No: 20);
(iv) FR1 (1st to 23rd amino acid residues of an amino acid sequence shown by SEQ ID No: 4, 1st to 23rd amino acid residues of an amino acid sequence shown by SEQ ID No: 8, 1st to 23rd amino acid residues of an amino acid sequence shown by SEQ ID No: 12, 1st to 23rd amino acid residues of an amino acid sequence shown by SEQ ID No: 16, 1st to 23rd amino acid residues of an amino acid sequence shown by SEQ ID No: 20);
(v) FR2 (35th to 49th amino acid residues of an amino acid sequence shown by SEQ ID No: 4, 40th to 54th amino acid residues of an amino acid sequence shown by SEQ ID No: 8, 41st to 55th amino acid residues of an amino acid sequence shown by SEQ ID No: 12, 35th to 49th amino acid residues of an amino acid sequence shown by SEQ ID No: 16 and 41st to 55th amino acid residues of an amino acid sequence shown by SEQ ID No: 20);
(vi) FR3 (57th to 88th amino acid residues of an amino acid sequence shown by SEQ ID No: 4, 61st to 93rd amino acid residues of an amino acid sequence shown by SEQ ID No: 8, 63rd to 94th amino acid residues of an amino acid sequence shown by SEQ ID No: 12, 57th to 88th amino acid residues of an amino acid sequence shown by SEQ ID No: 16 and 63rd to 94th amino acid residues of an amino acid sequence shown by SEQ ID No: 20);
(vii) FR4 (98th to 108th amino acid residues of an amino acid sequence shown by SEQ ID No: 4, 103rd to 113th amino acid residues of an amino acid sequence shown by SEQ ID No: 8, 103rd to 113th amino acid residues of an amino acid sequence shown by SEQ ID No: 12, 98th to 107th amino acid residues of an amino acid sequence shown by SEQ ID No: 16 and 103rd to 113th amino acid residues of an amino acid sequence shown by SEQ ID No: 20).

In another embodiment, the above-mentioned protein [b] is, for example, [b⁴] a protein having an amino acid sequence having significant homology to an amino acid sequence shown by SEQ ID No: 4, an amino acid sequence shown by SEQ ID No: 8, an amino acid sequence shown by SEQ ID No: 12, an amino acid sequence shown by SEQ ID No: 16, SEQ ID No: 20 or an amino acid sequence of the protein of [b¹] above, which binds to a female sex hormone after forming a complex with a protein having an amino acid sequence having significant homology to an amino acid sequence shown by SEQ ID No: 2, an amino acid sequence shown by SEQ ID No: 6, an amino acid sequence shown by SEQ ID No: 10, an amino acid sequence shown by SEQ ID No: 14, an amino acid sequence shown by SEQ ID No: 18 or an amino acid sequence of the protein of [a] above.

In the present invention, the number of amino acids deleted, substituted or added in the amino acid sequence shown by any SEQ ID NO: X is not subject to limitation, as long as it is 1 or more than 1, and can for example, be 1 to 80, preferably about 1 to 20, more preferably about 1 to 9, still more preferably 1 to 5, and most preferably several (1 or 2).

In the present invention, amino acid replacement is not subject to limitation, as long as a particular amino acid is substituted with an optionally chosen amino acid, and can, for example, be conservative amino acid substitution or non-conservative amino acid substitution. "Conservative amino acid substitution" refers to substituting a particular amino acid with an amino acid having a side chain of similar nature. Specifically, in conservative amino acid substitution, a particular amino acid is substituted with another amino acid belonging to the same group. On the other hand, "non-conservative amino acid substitution" refers to substituting a particular amino acid with another amino acid having a side chain of different nature. Specifically, in non-conservative amino acid substitution, a particular amino acid is substituted with another amino acid belonging to a different group. Groups of amino acids having a side chain of similar nature are known in the art. As such groups of amino acids, for example, amino acids having a basic (i.e., positively charged) side chain (e.g., lysine, arginine, histidine), amino acids having an acidic (i.e., negatively charged) side chain (e.g., aspartic acid, glutamic acid), and amino acids having a neutral (i.e., uncharged) side chain (e.g., glycine, asparagine, glutamine, serine, threonine, tyrosine, cysteine, alanine, valine, leucine, isoleucine, proline, phenylalanine, methionine, tryptophan). Amino acids having a neutral side chain can further be classified into amino acids having a polar side chain (e.g., glycine, asparagine, glutamine, serine, threonine, tyrosine, cysteine) and amino acids having a non-polar side chain (e.g., alanine, valine, leucine, isoleucine, proline, phenylalanine, methionine, tryptophan). Other groups can include, for example, amino acids having an aromatic side chain (e.g., phenylalanine, tryptophan, histidine), amino acids having a side chain containing a hydroxyl group (alcoholic hydroxyl group, phenolic hydroxyl group) (e.g., serine, threonine, tyrosine), and the like.

As amino acid sequences having significant homology to the amino acid sequence shown by any SEQ ID NO: X, for example, amino acid sequences having homology to the amino acid sequence shown by any SEQ ID NO: X of about 40% or more, preferably 60% or more, more preferably about 80% or more, still more preferably about 90% or more, and most preferably about 95% or more can be mentioned.

Degree of homology (%) can be determined by a method known *per se.* For example, degree of homology (%) can be determined using the Gap program employing the algorithm of Smith and Waterman (Adv. Appl. Math., 1981, 2, 482-489) (Wisconsin Sequence Analysis Package, Version 8 for Unix, Genetics Computer Group, University Research Park, Madison WI) in default settings. The BLAST program employing the algorithm of Karlin and Altschul (Proc. Natl. Acad. Sci. USA, 1990, 87:2264-2268, Proc. Natl. Acad. Sci. USA, 1993, 90:5873-5877) can also be used. For example, when comparing protein homology, degree of homology (%) can be determined using the XBLAST program in default settings. Furthermore, the ALIGN program (version 2.0) (a portion of GCG sequence alignment software package) employing the algorithm of Myers and Miller (CABIOS, 1988, 4:11-17) can also be used. As settings for comparing amino acid sequences using the ALIGN program, for example, "PAM120 weight residue table, gap length penalty = 12, gap penalty = 4" can be mentioned. These programs can also be used for determining degree of homology (%) of base sequences.

"Binding to a female sex hormone upon formation of a complex" means that the complex is reactive to the female sex hormone. The female sex hormone may be any one, as long as it binds to an estrogen receptor (e.g., estrogen receptor α, estrogen receptor β) and exhibits estrogen-like action, and is exemplified by estrogen and synthetic female sex hormones, with preference given to estradiol, estrone, estriol, ethynyl estradiol and diethylbestrol. Whether or not the complex is capable of binding to a female sex hormone can be determined by a method known per se such as the measuring method described below or a method based thereon. The complex of the present invention may be any one, as long as it is capable of binding to any of the aforementioned female sex hormones.

In an embodiment of the present invention, the proteins [a²]-[a⁴] or [b²]-[b⁴] above, which have varied binding capability or cross-reactivity for a female sex hormone, can be obtained by introducing a deletion, substitution or addition of 1 or more amino acids to a protein having the amino acid sequence shown by SEQ ID NO:2, the amino acid sequence shown by SEQ ID NO:4, the amino acid sequence shown by SEQ ID NO:6, the amino acid sequence shown by SEQ ID NO:8, the amino acid sequence shown by SEQ ID NO:10, the amino acid sequence shown by SEQ ID NO:12, the amino acid sequence shown by SEQ ID NO:14, the amino acid sequence shown by SEQ ID NO:16, the amino acid sequence shown by SEQ ID NO:18 or the amino acid sequence shown by SEQ ID NO:20, or to the protein [a¹] or [b¹]. In the proteins [a²]-[a⁴] or [b²]-[b⁴] above, the region to accept a deletion, substitution or addition of 1 or more amino acids can be 1 or more regions selected from the group consisting of CDR1, CDR2, CDR3, FR1, FR2, FR3, and FR4.

The partial peptide of the present invention may be any one, as long as it is a peptide that constitutes a portion of the protein [a] or [b] above; there may be used, for example, a peptide consisting of at least 6 or more, preferably at least 8 or more, more preferably at least 10 or more, still more preferably at least 12 or more, and most preferably 15 or more consecutive amino acids in the amino acid sequence of protein [a] or [b] above. As the partial peptide of the present invention, there can also be used a partial peptide having (or consisting of) an amino acid sequence corresponding to the CDR1, CDR2, CDR3, FR1, FR2, FR3 or FR4 of protein [a] or [b] above.

As salts of the protein of the present invention or partial peptide thereof, there may be used salts known per se, for example, acid adduct salts. These acid adduct salts include, for example, salts with inorganic acids (e.g., hydrochloric acid, phosphoric acid, hydrobromic acid, sulfuric acid) or salts with organic acids (e.g., acetic acid, formic acid, propionic acid, fumaric acid, maleic acid, succinic acid, tartaric acid, citric acid, malic acid, oxalic acid, benzoic acid, methanesulfonic acid and benzenesulfonic acid).

In the present invention, the "complex" may be any one, as long as protein [a] above and protein [b] above are joined together, and is exemplified by a complex wherein protein [a] above and protein [b] above are covalently bound in the presence or absence of a linker. This complex can also be used in the form of a salt (preferably an acid adduct salt) as with the aforementioned protein and partial peptide.

As the linker for fusing protein [a] above and protein [b] above, there may be used without limitation any linker known in the art; such linkers include, for example, peptides of repeated sequences of GGGS (SEQ ID NO:27), GSTSGSGKSSEGKG (SEQ ID NO:56), GSTSGSGKSSEGSGSTKG (SEQ ID NO:57), GSTSGKPSEGKG (SEQ ID NO:58), GSTSGSGKPGSGEGSTKG (SEQ ID NO:59), etc. [see, for example, Production of single-chain Fv monomers and multimers, D. Filpula, J. McGuire, and M. Whitlow. In "Antibody Engineering" edited by J. McCafferty, H. R. Hoogenboon, and D. J. Chiswell. pp.253-268, IRL PRESS (1996)]. A complex wherein protein [a] above and protein [b] above are covalently bound in the presence or absence of a linker can, for example, be obtained by separately preparing protein [a] above and protein [b] above, and subsequently covalently binding these proteins via a linker or by a direct covalent linkage. However, this method is painstaking because it requires a step to join protein [a] above and protein [b] above after their preparation, so as to obtain their complex. Another drawback resides in that a plurality of complexes with different covalent bond sites can result in, so that a single complex that is preferred from the viewpoint of reproducibility etc. is difficult to prepare. Therefore, the complex of the present invention is, for example, preferably a single-chain antibody (herein also referred to as recombinant antibody, single-stranded variable region antibody or single-stranded antibody, etc.) wherein protein [a] above and protein [b] above are fused by an amide linkage via a peptide linker or by a direct amide linkage. Such a single-chain antibody is useful in that it can easily be prepared from a transformant carrying an expression vector that harbors the base sequence encoding protein [a] above, the base sequence encoding a peptide linker (when obtaining a single-chain antibody amide-bound via a linker), and the base sequence encoding protein [b] above in frame. The base sequence encoding a peptide linker may be any one, as long as it does not contain stop codon in frame with the base sequences encoding proteins [a] and [b] above.

A peptide linker can be selected as appropriate by a method known in the art. Specifically, as a peptide linker, there may be used peptides of optionally chosen length consisting of 1 or more amino acid residues; there may be used, for example, peptides consisting of 10 or more amino acid residues.

The present invention also provides a polynucleotide (herein understood to be the same as gene and DNA) encoding the amino acid sequence of the protein of the present invention. The polynucleotide of the present invention may be any one, as long as it contains the aforementioned base sequence encoding the protein of the present invention.

Specifically, as the polynucleotide of the present invention, there may be mentioned base sequences encoding protein [a] above (e.g., base sequence shown by SEQ ID NO:1, SEQ ID NO:5, SEQ ID NO:9, SEQ ID NO:13 or SEQ ID NO:17), base sequences encoding protein [b] above (e.g., base sequence shown by SEQ ID NO:3, SEQ ID NO:7, SEQ ID NO:11, SEQ ID NO:15 or SEQ ID NO:19), and base sequences encoding the single-chain antibody above. As the polynucleotide of the present invention, there may also be mentioned a polynucleotide having the base sequence encoding a protein obtained by gene recombination of the protein of the present invention.

The present invention also provides a phage characterized by the expression of the single-chain antibody of the present invention. Although the phage of the present invention is not subject to limitation, as long as it expresses the single-chain antibody of the present invention, it is preferable that the single-chain antibody of the present invention be presented on the phage surface. Any phagemid can be used, as long as it is capable of presenting the single-chain antibody of the present invention on the phage surface; such phagemids include, for example, pCANTAB 5E (Amersham-Pharmacia Biotech), p8V5 (Affymax) and the like [see, for example, Vectors for Phage Display, N. Armstrong, N. B. Adey, S. J. McConnell, and B. K. Kay, in "Phage Display of Peptides and Proteins" edited by B. K. Kay, J. Winter, and J. McCafferty. pp.35-53, Academic Press (1996)]. Any helper phage can be used, as long as it is usable in a host transformed with the aforementioned phagemid; such helper phages include, for example, M13-series helper phages such as M13K07 (Amersham-Pharmacia Biotech).

The aforementioned gene (DNA, polynucleotide) of the present invention can be obtained using a method known *per se* according to the disclosure herein. For example, the gene of the present invention can be obtained from a hybridoma that produces an anti-female sex hormone monoclonal antibody, which is not to be construed as limitative. The N-terminal amino acid sequence of the antibody protein is determined, a primer having a base sequence deduced from this amino acid sequence is then prepared, mRNA is prepared from an antibody-producing hybridoma by a known method, and single-stranded cDNA is synthesized on the basis of the mRNA using reverse transcriptase, after which the gene of the present invention can be obtained selectively using PCR, hybridization, etc. on the basis of the amino acid sequence or base sequence of the variable region of a heavy chain or light chain of an anti-female sex hormone monoclonal antibody disclosed herein. Such methods are well-known; those skilled in the art can easily isolate the gene of the present invention on the basis of the disclosure herein. As specific procedures for these methods, there may be mentioned, for example, the method described in Molecular Cloning, 3rd edition (J. Sambrook et. al., Cold Spring Harbor Lab. Press, 2001). Useful methods of mRNA extraction include the method described in the operating manual for the Amersham QuickPrep mRNA purification kit; useful methods of cDNA synthesis and 5'-RACE include the methods described in the instruction manual for the CLONTECH Laboratories SMART RACE kit.

Regarding how to prepare recombinant antibodies, Chapter 2 of "RECOMBINANT ANTIBODIES" [edited by F. Breitling, John Wiley & Sons (USA), 1999] describes a method of preparing recombinant antibody fragments, a method of cloning antibody genes from hybridoma cell lines, a method of preparing antibody gene libraries, a method of selection of recombinant antibodies from gene libraries, a method of antibody engineering, etc., using which methods recombinant antibodies can be prepared.

Chapter 4 of the same book describes a method of producing recombinant antibodies and recombinant antibody fragments, and methods of their expression, including expression in rabbit reticulocyte lysate in vitro; expression in prokaryotes such as E. coli cytoplasm, soluble fraction of periplasm, inclusion body of periplasm, Bacillus and Streptomyces; and also described are methods of expression in eukaryotes such as Pichia, Saccharomyces, Schizosaccharomyces and other yeasts, Trichoderma and other fungi; expression in insect cells such as Baculovirus; expression in animal cells such as myeloma, CHO, and COS, transgenic plants such as of tobacco, and transgenic animals, using which methods transformants can be prepared.

Chapter 4 of the same book also describes methods of purifying recombinant antibodies and recombinant antibody fragments, wherein the desired product is first separated by a physical means, for example, cell harvest by centrifuging transformant, cell disruption by ultrasonication etc., mechanical milling, or enzymatic lysis. Subsequently, purification is conducted using a combination of ion exchange chromatography, size exclusion chromatography, thiophilic adsorption chromatography, affinity chromatography, etc. Affinity chromatography, in particular, is an efficient method; the desired product can be produced by purification using an antigen-specific method based on antigen recognition specificity, an antibody-specific method based on binding to the Fc or Fab' portion of protein A or protein G, or in the case of scFv without these portions, a method comprising expressing the ScFv as a fusion antibody having a small peptide fragment called "tag" and an affinity column specific for this tag is used (e.g., His-tag, c-myc tag, Strep tag, etc.).

First, a cDNA library of anti-female sex hormone monoclonal antibody-producing cells is constructed, and the cDNA library is then screened using a cDNA encoding the N-terminal sequence of the highly conservative constant region or the variable region of a heavy or light chain of an immunoglobulin as a probe; anti-female sex hormone monoclonal antibody light or heavy chain cDNA can be thus isolated.

As specific procedures for these methods, there may be mentioned, for example, the method described in Molecular Cloning, 3rd edition (J. Sambrook et. al., Cold Spring Harbor Lab. Press, 2001).

The gene of the present invention may also be synthesized chemically using a well-known technique on the basis of the sequence described herein.

Methods of gene recombination of the protein of the present invention include methods known *per se*; there may be used, for example, methods of converting the base sequence encoding the protein. Conversion of DNA base sequences can be achieved by a method known *per se* such as the ODA-LAPCR method, the Gupped duplex method, or the Kunkel method, or a method based thereon, using PCR or a known kit such as Mutan™-Super Express Km (Takara Shuzo Co., Ltd.) or Mutan™-K (Takara Shuzo Co., Ltd.) and the like. Depending on the purpose of use, the cloned antibody protein-encoding DNA can be used as is, or after being digested with a restriction enzyme or added with a linker as necessary. This DNA may have ATG as a translation initiation codon on the 5'-terminus thereof and TAA, TGA or TAG as a translation stop codon on the 3'-terminus thereof. These translation initiation codons and translation stop codons can also be added using an appropriate synthetic DNA adapter. An expression vector for the antibody protein of the present invention can, for example, be produced by (a) cleaving the desired DNA fragment from the DNA encoding the antibody protein of the present invention, and (b) joining the DNA fragment to downstream of a promoter in an appropriate expression vector.

### Methods of preparing recombinant antibodies

Various forms of recombinant antibodies can be prepared and are exemplified by those described in Roland Kontermann's ANTIBODY ENGINEERING HOME PAGE (http://aximt1.imt.unimarburg.de/-rek/AEP.html, February 25, 2002); for example, recombinant antibodies can be prepared in the form of Fab' fragments, F(ab') fragments, Fv fragments (Fv), single-chain Fv fragments (scFv), bispecific-chimeric scFV (χ-scFv), tandem scFV (scFv)2, bispecific-(scFv)2, disulfide-linked scFv, disulfide-stabilized Fv fragments (dsFv), diabody, single-chain diabody (scDb), bivalent diabody, bispecific diabody, knob-into-hole stabilized diabody, disulfide-stabilized diabody, triabody, tetrabody, trispecific triabody, CL-dimerized scFv, CH1-CL-dimerized scFv, CH3-dimerized scFv, knob-into-hole CH3-dimerized scFv, CH3-dimerized bivalent diabody, Fc-dimerized scFv, Fab-scFv fusions, Ig-scFv fusions, leucine-zipper stabilized scFv dimers, helix-stabilized scFv dimers, 4 helix-bunde stabilized scFv tetramers, streptavidin-scFv, and intrabody.

Methods wherein an antibody having a desired useful property is selected by shuffling mutated antibody genes also fall in the scope of the present invention.

### Recombinant antibody expression systems

Any recombinant antibody expression system can be used, as long as it is capable of efficiently expressing a recombinant antibody; for example, as tabulated in Roland Kontermann's ANTIBODY ENGINEERING HOME PAGE (http://aximt1.imt.uni-marburg.de/-rek/AEP.html, February 25, 2002), expression of Fv, scFv and scFv derivatives, bivalent and bispecific scFv, scFv or Fab-fusion proteins, intrabodies, etc. in mammalian cells, expression of scFV, Fab,.etc. in insect cells, expression of Fv, scFv, Fab, etc. in fungal cells, and expression of scFv in plant cells are known, and hence a variety of expression systems can be used.

### Methods of constructing a cDNA library

Any method of constructing a cDNA library can be used, as long as it is capable of efficiently preparing a cDNA library; such methods include the phage display method described in Roland Kontermann's ANTIBODY ENGINEERING HOME PAGE (http://aximt1.imt.uni-marburg.de/∼rek/AEP.htm1, February 25, 2002).

### Recombinant antibody selection methods

As useful methods of selecting the desired recombinant antibody from the library constructed, the methods described in Roland Kontermann's ANTIBODY ENGINEERING HOME PAGE (http://aximt1.imt.uni-marburg.de/-rek/AEP.html, February 25, 2002), such as the protocol "Isolation of Recombinant Antibodies from Phagemid Libraries" and "Isolation of Peptides from fd Phage Libraries" can be used.

The gene (DNA) used for expression may have substantially the same amino acid sequence as the anti-female sex hormone antibody protein expressed by the gene (DNA) obtained as described above, or a partial peptide thereof. Substantially the same amino acid sequence is exemplified by amino acid sequences with a partial modification (substitution), with an amino acid sequence added, or with an amino acid sequence removed (deleted).

Depending on the purpose of use, the gene (DNA) can be used as is, or after being cleaved or added with another DNA as necessary. This DNA may have a translation initiation codon ATG on the terminal thereof. This can be achieved by a method known *per se.* Such methods include, for example, the method described in Molecular Cloning, 3rd edition (J. Sambrook et. al., Cold Spring Harbor Lab. Press, 2001).

By incorporating the thus-obtained DNA (polynucleotide), a promoter, a translation initiation codon, the appropriate signal sequence, etc., by a method known *per se* , into a vector, a recombinant vector can be produced.

As such vectors, promoters and host strains, there may be mentioned, for example, the vectors, promoters and Escherichia strains described in Appendix 3 to Molecular Cloning, 3rd edition (J. Sambrook et. al., Cold Spring Harbor Lab. Press, 2001).

In addition to the aforementioned vectors, useful vectors include plasmids of *Escherichia coli* origin (pET-276, pCANTAP-5E, pUC19, pT7Bule T), plasmids of *Bacillus subtilis* origin (e.g., pUB110, pTP5, pC194), plasmids of yeast origin (e.g., pSH19, pSH15), λ phage, bacteriophages such as M13K07, and animal viruses such as retrovirus, vacciniavirus and vaculovirus, as well as pA1-11, pXT1, pRc/CMV, pRc/RSV, pcDNAI/Neo and the like.

Any promoter can be used, as long as it is suitable for the host used for gene expression. Preferred promoters include, for example, the trp promoter, the lac promoter, the recA promoter, the λP_{L} promoter, and the lpp promoter when the host is a bacterium of the genus *Escherichia;* the SPO1 promoter, the SPO2 promoter, and the penP promoter when the host is a bacterium of the genus *Bacillus;* and the PHO5 promoter, the PGK promoter, the GAP promoter, and the ADH promoter when the host is a yeast. When the host is an animal cell, the SRα promoter, the SV40 promoter, the LTR promoter, the CMV promoter, the HSV-TK promoter, etc. are preferred; when the host is an insect cell, the polyhedrin promoter, the P10 promoter, etc. are preferred.

In addition to the aforementioned promoters, the expression vector may contain an enhancer, splicing signal, poly A addition signal, selection marker, SV40 replication origin, etc. as necessary. Selection markers include, for example, the ampicillin resistance gene (hereinafter also referred to as Amp^{R}), the kanamycin resistance gene (hereinafter also referred to as Km^{R}), and the chloramphenicol resistance gene (hereinafter also referred to as Cm^{R}).

Where necessary, a signal sequence suitable for the host is added to the N-terminus side of the antibody protein of the present invention. Useful signal sequences include the phoA signal sequence, the ompA signal sequence and the like when the host is a bacterium of the genus *Escherichia;* the α-amylase signal sequence, the subtilisin signal sequence and the like when the host is a bacterium of the genus *Bacillus;* the MFα signal sequence, the SUC2 signal sequence and the like when the host is a yeast; and the insulin signal sequence, the α-interferon signal sequence, and the antibody molecule signal sequence and the like when the host is an animal cell. Using the thus-constructed vector harboring the DNA encoding the antibody protein of the present invention, a transformant can be produced.

Useful hosts include bacteria of the genus *Escherichia,* bacteria of the genus *Bacillus,* yeasts, insect cells, insects, animal cells and the like. Examples of useful bacteria of the genus *Escherichia* include *Escherichia coli* K12-DH1 [Proc. Natl. Acad. Sci. USA, vol. 60, 160 (1968)], JM103 [Nucleic Acids Research, vol. 9, 309 (1981)], JA221 [Journal of Molecular Biology, vol. 120, 517 (1978)], HB101 [Journal of Molecular Biology, vol. 41, 459 (1969)], C600 [Genetics, vol. 39, 440 (1954)], BL21DE3(pLysS), TG-1, and JM109. Useful bacteria of the genus *Bacillus* include, for example, *Bacillus subtilis* MI114 [Gene, vol. 24, 255 (1983)] and 207-21 [Journal of Biochemistry, vol. 95, 87 (1984)]. Useful yeasts include, for example, *Saccharomyces cerevisiae* AH22, AH22R-, NA87-11A, DKD-5D, and 20B-12, *Schizosaccharomyces pombe* NCYC1913 and NCYC2036, and *Pichia pastoris.* When the virus is AcNPV, useful insect cells include, for example, established *Spodoptera frugiperda* larva cells (Sf cells), *Trichoplusia ni* midgut MG1 cells, *Trichoplusia ni* egg High Five™ cells, *Mamestrabrassicae* cells, *Estigmena acrea* cells and the like. When the virus is BmNPV, silkworm-derived established cells *(Bombyx mori* N; BmN cells) etc. are used. Such Sf cells include, for example, Sf9 cells (ATCC CRL1711) and Sf21 cells [both described by Vaughn, J.L. et al. In Vivo, 13, 213-217 (1977)]. Useful insects include, for example, silkworm larvae [Maeda et al., Nature, vol. 315, 592 (1985)]. Useful animal cells include, for example, simian COS-7 cells, Vero, Chinese hamster CHO cells, mouse L cells, mouse AtT-20, mouse myeloma cells, rat GH3 and human FL cells.

Transformation of bacteria of the genus Escherichia can, for example, be achieved according to the methods described in Proc. Natl. Acad. Sci. USA, vol. 69, 2110 (1972) and Gene, vol. 17, 107 (1982). Transformation of bacteria of the genus *Bacillus* can, for example, be achieved according to the method described in Molecular & General Genetics, vol. 168, 111 (1979). Transformation of yeasts can, for example, be achieved according to the methods described in Methods in Enzymology, vol. 194, 182-187 (1991) and Proc. Natl. Acad. Sci. USA, vol. 75, 1929 (1978). Transformation of insect cells or insects can, for example, be achieved according to the method described in Bio/Technology, vol. 6, 47-55 (1988). Transformation of an animal cell can, for example, be achieved using the method described in SAIBO KOGAKU (CELL TECHNOLOGY), Supplementary 8: Shin Saibou Kougaku Jikken Purotokoru, 263-267 (1995) (published by Shujunsha Co., Ltd.) and Virology, Vol. 52, 456 (1973).

Thus, a transformant transformed with an expression vector harboring the DNA encoding an antibody protein is obtained.

Furthermore, the protein of the present invention can be produced by cultivating the thus-obtained transformant and harvesting the resulting protein.

Regarding culture media, liquid media are suitable for the cultivation of a transformant whose host is a bacterium of the genus *Escherichia* or *Bacillus,* which media are supplemented with carbon sources, nitrogen sources, minerals, and other substances required for the growth of the transformant. Carbon sources include, for example, glucose, dextrin, soluble starch, sucrose and the like; nitrogen sources include, for example, inorganic or organic substances such as ammonium salts, nitrates, corn steep liquor, peptone, casein, meat extract, soybean flour, potato extract and the like; minerals include, for example, calcium chloride, sodium dihydrogen phosphate, magnesium chloride and the like. Yeast, vitamins, growth promoters, etc. may also be added. It is desirable that the pH of the medium be about 5 to 8.

Preferred media for cultivating bacteria of the genus *Escherichia* include, for example, M9 medium supplemented with glucose and casamino acid [Miller, Journal of Experiments in Molecular Genetics, 431-433, Cold Spring Harbor Laboratory, New York (1972)]. To increase promoter efficiency where necessary, an agent like 3β-indolylacrylic acid or isopropylthiogalactoside (IPTG), for example, may be added. When the host is a bacterium of the genus *Escherichia,* cultivation is normally carried out at about 15 to 43°C for about 3 to 24 hours, and aeration and stirring may be conducted as necessary. When the host is a bacterium of the genus *Bacillus,* cultivation is normally carried out at about 30 to 40°C for about 6 to 24 hours, and aeration and stirring may be conducted as necessary. Media for cultivating a transformant whose host is a yeast include, for example, Burkholder's minimum medium [Bostian, K.L. et al., Proc. Natl. Acad. Sci. USA, vol. 77, 4505 (1980)] and SD medium supplemented with 0.5% casamino acid [Bitter, G.A. et al., Proc. Natl. Acad. Sci. USA, vol. 81, 5330 (1984)]. It is preferable that the medium's pH be adjusted to about 5 to 8. Cultivation is normally carried out at about 20 to 35°C for about 24 to 72 hours, with aeration and stirring conducted as necessary.

Useful media for cultivating a transformant whose host is an insect cell or an insect include Grace's insect medium [Grace, T.C.C., Nature, 195, 788 (1962)] supplemented with additives such as inactivated 10% bovine serum as appropriate. It is preferable that the medium's pH be adjusted to about 6.2 to 6.4. Cultivation is normally carried out at about 27°C for about 3 to 5 days, with aeration and stirring conducted as necessary. Useful media for cultivating a transformant whose host is an animal cell include, for example, MEM medium supplemented with about 5 to 20% fetal bovine serum [Science, vol. 122, 501 (1952)], DMEM medium [Virology, vol. 8, 396 (1959)], RPMI1640 medium [The Journal of the American Medical Association], vol. 199, 519 (1967)], and 199 medium [Proceeding of the Society for the Biological Medicine, vol. 73, 1 (1950)]. It is preferable that pH be about 6 to 8. Cultivation is normally carried out at about 30 to 40°C for about 15 to 60 hours, with aeration and stirring conducted as necessary. As described above, the antibody protein of the present invention can be produced in the cells or cell membrane of the transformant or outside the cells.

Separation and purification of the desired antibody protein of the present invention from the thus-obtained culture can, for example, be achieved by the method described below. When extracting the antibody protein of the present invention from cultured cells, there may be used as appropriate, for example, a method wherein cultured cells are collected by a known method, suspended in an appropriate buffer solution, and disrupted by means of ultrasound, lysozyme and/or freeze-thawing etc., after which a crude extract of antibody protein is obtained by centrifugation or filtration. The buffer solution may contain a protein denaturant such as urea or guanidine hydrochloride and a surfactant such as Triton X-100™. If the antibody protein is secreted in the culture broth, it is treated by a method known *per* se to separate the cells and the supernatant after completion of cultivation and the supernatant is collected. Purification of the antibody protein contained in the thus-obtained culture supernatant or extract can be achieved by appropriately combining publicly known methods of separation and purification. Useful known methods of separation and purification include methods based on solubility, such as salting-out and solvent precipitation; methods based mainly on molecular weight differences, such as dialysis, ultrafiltration, gel filtration, and SDS-polyacrylamide gel electrophoresis; methods based on charge differences, such as ion exchange chromatography; methods based on specific affinity, such as affinity chromatography; methods based on hydrophobicity differences, such as reversed-phase high performance liquid chromatography; and methods based on isoelectric point differences, such as isoelectric focusing.

The protein of the present invention or a salt thereof can be produced by a method known *per se* of protein synthesis, or by cleaving the protein of the present invention using an appropriate protease. This protein synthesis can, for example, be achieved by solid phase synthesis or liquid phase synthesis. Specifically, the desired protein can be produced by condensing the partial peptide or amino acids that constitute the protein of the present invention and the remainder, and if the purified product has a protective group, removing the protective group. Known methods of condensation and the methods of protective group removal described below, for example, can be used.
(1) M. Bodanszky and M.A. Ondetti, Peptide Synthesis, Interscience Publishers, New York (1966)
(2) Schroeder and Luebke, The peptide, Academic Press, New York (1965)
(3) Nobuo Izumiya et al., *Peputido Dogei no Kiso to Jikken,* Maruzen Co., Ltd. (1975)
(4) Haruaki Yajima and Shunpei Sakakibara, *Seikagaku Jikken Kouza* 1, Tanpakushitsu no Kagaku IV, 205, (1977)
(5) Haruaki Yajima, supervisor, *Zoku Iyakuhin no Kaihatsu,* Vol. 14: *Peputido Gosei,* Hirokawa Publishing

After the reaction, the protein of the present invention can be purified and isolated using ordinary purification methods such as solvent extraction, distillation, column chromatography, liquid chromatography, and recrystallization in combination. If the protein obtained by the method described above is a free from, it can be converted to an appropriate salt by a known method; if the protein obtained is a salt, it can be converted to a free form by a known method.

The thus-obtained antibody, complex, protein (anti-female sex hormone antibody recombinant protein) and/or phage of the present invention can be used as a reagent for quantitatively measuring environmental hormones, or can be used to produce affinity columns for concentrating environmental hormones, in which it is immobilized to various carriers. Additionally, by identifying a female sex hormones that binds (i.e., cross-reacts) to the antibody, complex, protein and/or phage of the present invention, the applicability of the antibody, complex and/or protein of the present invention can be expanded. Furthermore, the present invention provides a kit for measuring or quantifying a female sex hormone containing the antibody, complex and/or protein of the present invention and a kit for concentrating a female sex hormone.

Although the aforementioned kit may contain only one kind of the antibody, complex, protein and/or phage of the present invention, it may also contain plural kinds of antibody, complex, protein and/or phage of the present invention. For example, by using a kit containing a plurality of complexes of different degrees of cross-reactivity, a particular female sex hormone can be measured or quantified with specificity. Specifically, to specifically measure and quantify a selected female sex hormone of particular characteristics in a sample containing a plurality of female sex hormones, a complex that binds to the female sex hormone and another complex that does not substantially bind to the female sex hormone may be used in combination (it is preferable, however, that these complexes are similar in terms of cross-reactivity to the other female sex hormones contained in the sample). The present invention also provides such a method.

As methods of measuring female sex hormones using the antibody, complex, protein and/or phage of the present invention, there may be mentioned various methods in common use for antigen detection, such as radioisotope immunoassay (RIA), ELISA [Engvall, E., Methods in Enzymol., 70, 419-439 (1980)], fluorescent antibody method, plaque method, spot method, agglutination method, and Ouchterlony method ("Hybridoma Method and Monoclonal Antibodies", published by R&D Planning, pages 30-53, March 5, 1982). From the viewpoint of sensitivity, simplicity, etc., ELISA is commonly used.

As a carrier for the antibody, protein and/or phage of the present invention, there may be mentioned, for example, microplates (e.g., 96-well microplate, 24-well microplate, 192-well microplate, 384-well microplate, etc.), test tubes (e.g., glass test tubes, plastic test tubes), glass particles, polystyrene particles, modified polystyrene particles, polyvinyl particles, latexes (e.g., polystyrene latex), nitrocellulose membranes, cyanogen bromide-activated filter paper, DBM-activated filter paper, granular solid phases (e.g., Sepharose, Sephadex, agarose, cellulose, Sephacryl, etc.), iron-containing polycarbonate films, and magnet-containing beads.

The antibody, complex, protein and/or phage of the present invention can be carried on carriers by methods known *per* se [e.g., "Enzyme Immunoassay" above, pp. 268∼296, "Affinity Chromatography Handbook", Amersham-Pharmacia Biotech K.K., published December 20, 1998].

In the immunological concentration method of the present invention, the desired substance of minimal immunological impurity contents can be concentrated at rates as high as several thousands to several tens of thousands of times, by passing a large amount of sample through a column of immunological adsorbent or mixing with immunological adsorbent particles to adsorb the desired female sex hormone, degradation products thereof or a mixture thereof to the immunological adsorbent by an antigen-antibody reaction, and subsequently eluting the desired product by known methods, such as changing the pH (lowering to pH 2.5-3, raising to pH 11.5), changing the ionic strength (1M NaCl etc.), changing the polarity [10% dioxane, 50% ethylene glycol, 3M chaotropic salts (SCN⁻, CCl₃COO⁻, I⁻ ), etc.], adding protein denaturants (8M urea, 6M guanidine hydrochloride, etc.), and conducting electrophoretic dissociation.

Female sex hormones occurring in very trace amounts in the environment, degradation products thereof, or a mixture thereof can thereby be concentrated at much higher rates than by conventional methods of concentration such as solvent extraction and solid phase extraction and in addition concentrates of lower contents of impurities and other substances that interfere with quantitation can be obtained.

When bases, amino acids and the like are shown in abbreviations in the present specification and Figures, they are based on the abbreviations by IUPAC-IUB Commission on Biochemical Nomenclature or abbreviations conventionally used in the pertinent field. Examples thereof are given in the following. When an amino acid can have an optical isomer, it is an L form, unless particularly indicated.
DNA :deoxy ribonucleic acid
cDNA :complementary deoxyribonucleic acid
a:A :Adenine
t:T :thymine
g:G :guanine
c:C :cytosine
x : unidentified
RNA :ribonucleic acid
mRNA :messenger ribonucleic acid
Abbreviations of amino acids
3 letters:1 letter:Japanese name
Gly : G :glycine
Ala : A :alanine
Val : V :valine
Leu : L :leucine
Ile : I :isoleucine
Ser : S :serine
Thr : T :threonine
Cys : C :cysteine
Met : M :methionine
Glu : E :glutamic acid
Asp : D :aspartic acid
Lys : K :lysine
Arg : R :arginine
His : H :histidine
Phe : F :phenylalanine
Tyr : Y :tyrosine
Trp : W :tryptophan
Pro : P :proline
Asn : N :asparagine
Gln . Q :glutamine
Asx : B :Asn+Asp
Glx : Z :Gln+Glu
Xaa :unknown or other amino acid

### Examples

The present invention is explained in more detail by illustrating Examples in the following, which are not to be construed as limitative.

The mouse hybridoma cells produced in the following Example 1 have been deposited at National Institute of Advanced Industrial Science and Technology, International Patent Organism Depository, Central 6, 1-1-1 Higashi, Tsukuba-shi, Ibaraki, Japan under the Budapest Treaty. Their deposit numbers and the date of deposit are shown in the following.
Mouse-hybridoma E2-73:
Deposit number: FERM BP-7569
Date of deposit: April 25, 2001
Mouse-hybridoma E1-420:
Deposit number: FERM BP-7568
Date of deposit: April 25, 2001
Mouse-hybridoma EE2-227:
Deposit number: FERM BP-7567
Date of deposit: April 25, 2001

In addition, the *Escherichia coli* transformant produced in the following Example 5 has been deposited at National Institute of Advanced Industrial Science and Technology, International Patent Organism Depository, Central 6, 1-1-1 Higashi, Tsukuba-shi, Ibaraki, Japan under the Budapest Treaty. The deposit numbers and the date of deposit are shown in the following.
Escherichia coli TG/pCAN-E2scFvHL:
Deposit number: FERM BP-7912
Date of deposit: February 22, 2002

### [Reference Example 1]

### 1. Acquisition of monoclonal antibodies

### 1?1 Production of hapten

### (1) Production of hapten for anti-ethynyl estradiol antibody

Ethynyl estradiol (EE2; 1.0 g) and sodium methylate (0.76 g) were dissolved in ethanol (35 ml). After adding monochloracetic acid (0.41g), the solution was refluxed under heating for 22 hr. After concentration under reduced pressure, water and ethyl acetate (about 100 ml each) were added to partition the solution. The aqueous layer was washed with ethyl acetate (50 ml) and acidified (pH 1-2) with conc. hydrochloric acid. After extracting with ethyl acetate (100 ml and 50 ml), the organic layer was washed with saturated brine (30 ml) and dried over anhydrous sodium sulfate. The residue was concentrated under reduced pressure and stood at -20°C for 2 days to allow a part thereof to be crystallized. This part was taken and used as a seed crystal. The concentrated solution was dissolved in a small amount of acetone, and hexane and the seed crystal were added to allow precipitation of crystals. The crystals were collected by filtration and vacuum dried to yield an object product, EE2-3-carboxymethylether (EE2-3CME).

### (2) Production of hapten for anti-17β-estradiol antibody

17β-Estradiol (E2; 1.0 g) and sodium methylate (0.82 g) were dissolved in ethanol (35 ml). After adding monochloracetic acid (0.45 g), the solution was refluxed under heating for 2 days. After concentration under reduced pressure, water (about 300 ml) and ethyl acetate (about 200 ml) was added to partition the mixture. The aqueous layer was washed with ethyl acetate (50 ml) and acidified (pH 1-2) with concentrated hydrochloric acid. After extracting twice with ethyl acetate (100 ml), the organic layer was washed with saturated brine (30 ml) and dried over anhydrous sodium sulfate. The residue was concentrated under reduced pressure and dissolved in a small amount of acetone. Isopropyl ether (IPE) was added to allow precipitation of crystals. The crystals were collected by filtration, washed with IPE and vacuum dried to yield an object product, E2-3-carboxymethylether (E2-3CME).

### (3) Production of hapten for estrone antibody

Estradiol-3,17-diacetate (E2-3,17-diAce, 20 g) was dissolved in acetic acid (25 ml). While cooling the solution, 4/5 of a mixture of anhydrous chromic acid (9 g), acetic acid (27 ml) and water (4 ml) was gradually added. After stirring overnight, water was added and the mixture was extracted with chloroform, washed with potassium carbonate solution, dried over sodium sulfate and concentrated under reduced pressure. The residue was dissolved in benzene, applied to column chromatography (Wako gel 2w) and eluted with hot benzene to give crude 6-oxo-E2-3,17-diAce (starting material was removed while confirming by TLC). This substance was dissolved in 2-methoxy-ethanol, the same amount of 20% NaOH was added and reacted at 100°C for 1 hr. After acidifying with dilute hydrochloric acid, the reaction mixture was eluted with ethyl acetate, dried over sodium sulfate, and concentrated under reduced pressure to allow recrystallization (6oxo-E2) from methanol. 6oxo-E2 (300 mg) was dissolved in ethanol (2 ml) and potassium carbonate fine powder (50 mg) was added. Benzyl bromide (50 µl) was added, the mixture was stirred (70°C, 2 hr) and concentrated, which was recrystallized from methanol (6-oxo- E2-3-benzyl ether). 6-oxo-E2-3-benzyl ether was dissolved in methanol, and carboxymethoxylamine?1/2 HCl and sodium methoxide (1.2 molar amount) were dissolved in a small amount of water, diluted with methanol and added. After reacting at room temperature for 1 hr, 2/3 was evaporated, and the resulting solution was acidified with water and HCl, extracted with ethyl acetate ion, and dried over sodium sulfate. The residue was dissolved in hexane, purified by silica gel column eluting with methanol and recrystallized from methanol. A recrystallized substance (0.6 g) was dissolved in acetone (2 ml) and a half of a mixture of anhydrous chromic acid (1 g), acetic acid (5 ml) and water (1 ml) was added. After generation of heat, the mixture was extracted with ethyl acetate and evaporated to dryness (6-oxo-E1-3-benzyl ether). 6-Oxo-E1-3-benzyl ether (400 mg) was dissolved in methanol (5 ml), 5% Pd?C (100 mg) was added and the mixture was stirred while blowing hydrogen. After reacting for 1 hr, the reaction mixture was evaporated to dryness, which was recrystallized from methanol to yield an object product, 6-oxo-E1-6carboxymethyloxime (E1-6CMO).

### 1-2 Preparation of immunogen

For each of 3 kinds of haptens obtained in 1-1, hapten (0.1 mol), water-soluble carbodiimide (0.14 mol) and N-hydroxysuccinimide (0.14 mol) were reacted in dimethyl sulfoxide (2 ml) overnight to produce an activated ester. Then, keyhole limpet hemocyanin (KLH; 10 mg) was dissolved in 0.13 M sodium bicarbonate (NaHCO₃) solution. After adding said activated ester (200 µl), the mixture was reacted at 4°C overnight. Unreacted reagents were removed by dialysis against Dulbecco's phosphate buffer (PBS), and the resultant was stored in a frozen state as immunogen.

### 1-3 immunization

The immunogen obtained in 1-2 was dissolved in PBS to be at concentration of 500 µg/ml, and added to an equal amount of Freund's adjuvant or RIBI adjuvant system. The mixture was sufficiently emulsified, and subcutaneously administered to a BALB/C mouse (female) at 100 µg/mouse. Additional immunizations were performed at 2 weeks (Freund's) or 3 weeks (RIBI) interval. After 5-6 additional immunizations, the immunogen (50 µg/0.1 ml PBS/mouse) was intravenously administered to the individual showing the highest serum antibody titer as final immunization.

### 1-4 Cell fusion

A spleen was removed from the mouse in which the final immunization was performed in 1-3 (3 days after the final immunization), and spleen cells were prepared. Separately cultured mouse myeloma cells and the spleen cells were contacted in the proportion of 1:5 in the presence of polyethylene glycol (average molecular weight 4,000) and carried cell fusion to give fused cells (hybridoma). These hybridomas were suspended in HAT medium, plated into 96-well microplate and cultured in a carbonic acid gas incubator (37°C, 5% CO₂) .

### 1-5 Screening of hybridomas

### (1) Production of assay plate

A goat anti-mouse IgAGM antibody (manufactured by ICN/Cappel, #55461) was dissolved in PBS at concentration of 50 µg/ml, and added into microplate at concentration of 100 µl/well. The plate was reacted at 4°C overnight, and washed with PBS containing 0.05% Tween20 (T-PBS) (300 µl/well, twice). Then, BlockAce (Snow Brand Milk Products Co., Tokyo) 4-fold diluted with PBS was added (200 µl/well). The plate was reacted at 4°C overnight or more, and cold-stored until it was used.

### (2) Production of antigen-enzyme conjugate (labeled antigen)

For each of 3 kinds of haptens produced in 1-1, hapten (0.1 mol), water-soluble carbodiimide (0.14 mol) and N-hydroxysuccinimide (0.14 mol) were reacted in dimethyl sulfoxide (2 ml) overnight to produce an activated ester. Then, a horseradish peroxydase (HRP; 10 mg) was dissolved in 0.13 M sodium bicarbonate (NaHCO₃) solution (10 ml), said activated ester (15 µl) was added, and the mixture was reacted at 4°C overnight. Unreacted reagents were removed by ultrafiltration, and the resultant was cold-stored at concentration of 3 mg/ml.

### (3) Primary screening (antigen binding ability test)

In the microplate into which the cells were plated in 1-4, the culture solution (100 µl) in the well in which cell growth was confirmed was added to the assay plate produced in (1) (after washing with PBS (or T-PBS) (300 µl/well, twice)). The plate was reacted at room temperature for 1 hr, and washed with T-PBS (300 µl/well, 3 times). The labeled antigen against the objective antibody produced in 1-5(2) was 5000-fold diluted with T-PBS, and added to the microplate. The plate was reacted at room temperature for 1 hr, and washed with T-PBS (300 µl/well, 3 times). TMB peroxydase substrate kit (Bio-Rad Japan, Tokyo, #172-1066: hereinafter, chromogeic substrate) (100 µl/well) was added, and the mixture was reacted at room temperature for 30 min. Then, 1 N phosphoric acid (100 µl/well) was added to stop the chromogenic reaction. The absorbance at wavelength of 450 nm was read off. The cells showing absorbance over 1 were scaled-up to 24-well microplate.

### (4) Secondary screening (female sex hormone inhibitory test)

For the cells scaled-up to 24-well microplate in 1-5(3), the culture solution (100 µl each) in the well in which sufficient cell growth was confirmed was added into plural wells of the assay plate produced in (1) (after washing with PBS (or T-PBS) (300 µl/well, twice)). The plate was reacted at room temperature for 1 hr, and washed with T-PBS (300 µl/well, 3 times). The measuring object female sex hormone (1 ng/ml in 10% methanol (hereinafter, MeOH)) or 10% MeOH alone (control) and the labeled antigen against the objective antibody (5000-fold diluted with T-PBS) were mixed in equal amounts and added to the microplate. The plate was reacted at room temperature for 1 hr, and washed with T-PBS (300 µl/well, 3 times). The chromogeic substrate) (100 µl/well) was added, and the mixture was reacted at room temperature for 30 min. Then, 1 N phosphoric acid (100 µl/well) was added to stop the chromogenic reaction. The absorbance at wavelength of 450 nm was read off. A cloning was carried out for the cells that reduction of absorbance by not less than 20% in comparison to the control, in the presence of female sex hormone, was confirmed according to a conventional method, resulting in obtaining hybridoma lines producing the objective antibody as follows.
(1) Anti-estradiol monoclonal antibody-producing mouse hybridoma cell
   E2-73 line (FERM BP-7569),
(2) Anti-estrone monoclonal antibody-producing mouse hybridoma cell
   E1-420 line (FERM BP-7568),
(3) Anti-ethynyl estradiol monoclonal antibody-producing mouse hybridoma cell
   EE2-227 line (FERM BP-7567),
(4) Anti-ethynyl estradiol monoclonal antibody-producing mouse hybridoma cell
   EE2-3 line
   (The resistance to a detergent of the antibody obtained from this line is the same as that of one from EE2-277 line.)
(5) Ethynyl estradiol anti-monoclonal antibody producing mouse hybridoma cell
   EE2-8-18 line
   (The resistance to a detergent of the antibody obtained from this line is slightly lower than that of one from EE2-277 line.)

The above-mentioned mouse hybridoma cell E2-73 line (FERM BP-7569), mouse hybridoma cell E1-420 line (FERM BP-7568), and mouse hybridoma cell EE2-227 line (FERM BP-7567) have been deposited at National Institute of Advanced Industrial Science and Technology, International Patent Organism Depository since April 25, 2001 with the above-mentioned deposit numbers.

### [Example 1] Determination of N-terminal amino acid sequence of anti-estradiol monoclonal antibody

The antibody protein purified from the culture supernatant of anti-estradiol monoclonal antibody-producing mouse hybridoma cells (E2-73 line) (FERM BP-7569) using Protein A column (PROSEP-A, purchased from Air Brown Co.) was separated and purified to heavy chain and light chain by SDS-polyacrylamide gel electrophoresis, and transferred onto PVDF membrane. After staining with Ponso-S, protein bands were excised. The excised proteins on the membrane were treated with Pfu Pyroglutamate Aminopeptidase (purchased from Takara Shuzo Co.) (2 mU) at 50°C for 17 hr, and their N-terminal amino acid sequences were determined with gas phase protein sequencer (Model 494 Applied Biosystems Japan). The following results were obtained.

E2-73 heavy chain: VQLQQSGAELARPGASVKLS (SEQ ID NO: 21)

E2-73 light chain: DIVMTQSPTFLSTSLGDKIT (SEQ ID NO: 22)

### [Example 2] Purification of anti-estradiol monoclonal antibody mRNA

Anti-estradiol monoclonal antibody producing mouse hybridoma cells (E2-73 line) (FERM BP-7569) (1X10⁷ cells/ml) were suspended in RNA preparation solution (ISOGEN solution, purchased from Wako Pure Chemical Industries, Ltd.) (1 ml) and disrupted, and RNA was extracted from the cells according to the manual. The concentration of RNA was determined from absorbance at 260nm. From the obtained RNA (about 100 µg), mRNA (about 1.5 µg) was obtained using mRNA purified kit (Oligotex-dT30 <super> mRNA Purification kit, purchased from Takara Shuzo Co.).

### [Example 3] Acquisition of cDNA fragments of anti-estradiol monoclonal antibody heavy chain and light chain variable regions and determination of their base sequences as well as deduction of amino acid sequences of heavy chain and light chain variable regions

The synthesis of cDNAs was carried out using about 100 ng of mRNA each for light chain and heavy chain, with First Strand cDNA Synthesis Kit ReverTra Ace -α™ (purchased from Toyobo Co.). Then, amplification of each cDNA fragment was carried out by PCR reaction in Personal cycler (purchased from Biometra) (94°C 30 sec, 55°C 30 sec, 72°C 2 min; Repeat number: 30 cycles), using primers for heavy chain and primers for light chain shown in the following, which were devised from their N terminal amino acid sequences obtained in Example 1, and Pfu Turbo (purchased from Toyobo Co.) enzyme.
Primers for E2-73 heavy chain <B: C, G, T> 
Forward 5′- CTATGCGGCCCAGCCGGCCATGGTBCAGCTGCAGCAGTCTGG -3′
(SEQ ID NO: 23)
 Back 5'- ACCACTCTCACAGTCTCCTCGG -3' (SEQ ID NO: 24) Primes for E2-73 light chain <Y: C, T>

Forward 5'- CTCAGTCTCCAGAYATCGTGATGACYCAGTCT -3' (SEQ ID NO:
25)

Back 5′- GGGACCAAGCTGGAAATAAAACGGA -3′ (SEQ ID NO: 26)

Thus, cDNA fragments for variable regions of heavy chain and light chain of anti-estradiol monoclonal antibody were obtained. Each fragment was purified from 2% agarose after electrophoresis using a micro spin column (purchased from Amersham Pharmacia Biotech Co.). Then, using dideoxy method, the base sequence of each variable region cDNA was determined, and the amino acid sequence was deduced therefrom. The base sequences were determined by DNA sequencer 310 (Applied Biosystems Japan), after the reaction in a Personal cycler (purchased from Biometra) with Big Dye Terminator Cycle Sequencing Ready Reaction Kit (purchased from Applied Biosystems Japan) using the above-mentioned PCR primers as sequencing primers.

### [Example 4] Conversion of anti-estradiol monoclonal antibody heavy chain and light chain variable region cDNAs to single chain (single chain variable region antibody; scFv) and construction of plasmid containing scFv fragment

The obtained fragments of heavy chain and light chain were ligated with a peptide linker ((GGGGS)X3) (SEQ ID NO: 27) to give a single chain (scFv). The scFv DNA fragment was ligated to pUC19 cut with *Hinc*II using a ligation kit (purchased from Takara shuzo). After the reaction, the mixture was added to JM109 competent cells (purchased from Toyobo) to allow transformation. The plasmid was purified from the transformant, the complete base sequence of the cDNA insert was determined using dideoxy method. Then, the identity to the base sequences of the above-mentioned variable regions was confirmed (SEQ ID NOs: 1 and 3), and the amino acid sequences were determined (SEQ ID NOs: 2 and 4).

Namely, SEQ ID NO: 1 depicts the base sequence of the heavy chain variable region gene of the antibody produced by mouse hybridoma cell line E2-73, SEQ ID NO: 2 depicts the amino acid sequence corresponding thereto.

SEQ ID NO: 3 depicts the base sequence of the light chain variable region gene of the antibody produced by mouse hybridoma cell line E2-73, SEQ ID NO: 4 depicts the amino acid sequence corresponding thereto.

### [Example 5] Expression of anti-estradiol monoclonal antibody variable region

A recombinant phage production system (purchased from Amersham Pharmacia Biotech) was used to express the cDNA of the anti-estradiol monoclonal antibody variable region. To utilize this system, the scFv, which was excised from the above-mentioned plasmid with restriction enzymes *Sfi*I and *Not*I and pCANTAB 5E phagemid (purchased from Amersham Pharmacia Biotech) were ligated using Quick Ligation Kit (purchased from NEB) to yield a plasmid pCANTAB-5E/E2-73scFvH. TG1 competent cells *(Escherichia coli* TG-1) (purchased from Toyobo) were transformed with the obtained plasmid by electroporation to produce a transformant *Escherichia* coli TG/pCAN-E2scFvHL harboring the plasmid pCANTAB-5E/E2-73scFvHL.

Furthermore, an antibody expressing phage was obtained using the transformant obtained in this way and a helper phage M13K07 (purchased from Amersham Pharmacia Biotech). Then, the reactivity of the scFv expressed on this phage against estradiol was confirmed by enzyme immunoassay (ELISA). In ELISA, at first, a hapten-protein complex (β-Estradiol 6-(o-carboxy-methyl)oxime:BSA; purchased from SIGMA) (100 µl) was added to a plate, and stood at 4°C overnight. Then, blocking with 3% skim milk was carried out at 37°C for 1.5 hr. The plate was washed with PBS twice. The expressed scFv was mixed with a free antigen (final concentration 1 mg/ml, 0.037 mg/ml or 0 mg/ml) and the mixture (100 µl) was added to the plate and stood at room temperature for 3 hr. Then, the plate was washed with 0.5% Tween/PBS (twice) and PBS (once), 500-fold diluted HRP/Anti-M13: secondary antibody (purchased from Amersham Pharmacia Biotech) (100 µl) was added, and the mixture was stood at room temperature for 2 hr. Then, the plate was washed with 0.5% Tween/PBS (twice) and PBS (once), a substrate solution (0.2% o-phenylenediamine solution, 0.003% H₂O₂ was added and the mixture was reacted at room temperature for 20 min. A reaction termination solution (4N H₂SO₄) was added and the absorbance (492 nm) was measured (Fig. 1). The obtained results confirmed that this scFv has a binding ability to a female sex hormone.

### [Example 6] Determination of N terminal amino acid sequence of anti-estrone monoclonal antibody

The antibody protein purified from the culture supernatant of anti-estrone monoclonal antibody-producing mouse hybridoma cells (E1-420 line) (FERM BP-7568) using Protein A column (PROSEP-A, purchased from Air Brown Co.) was separated and purified to heavy chain and light chain by SDS-polyacrylamide gel electrophoresis, and transferred onto PVDF membrane. After staining with Ponso-S, protein bands were excised. The excised proteins on the membrane were treated with Pfu Pyroglutamate Aminopeptidase (purchased from Takara Shuzo Co.) (2 mU) at 50°C for 17 hr, and N terminal amino acid sequences were determined with gas phase protein sequencer (Model 494 Applied Biosystems Japan). The following results were obtained.

E1-420 heavy chain: IQLVESGPELKKPGETVKIS (SEQ ID NO: 28)

E1-420 light chain: DVLMTQTPLTLSVTIGQPA (SEQ ID NO: 29)

### [Example 7] Purification of anti-estrone monoclonal antibody mRNA

Anti-estrone monoclonal antibody producing mouse hybridoma cell (E1-420 line) (FERM BP-7568) (1X10⁷ cells/ml) were suspended in RNA preparation solution (ISOGEN solution, purchased from Wako Pure Chemical Industries, Ltd.) (1 ml) and disrupted, and RNA was extracted from the cells according to the manual. The concentration of RNA was determined from absorbance at 260nm. From the obtained RNA (about 100 µg), mRNA (about 1.5µg) was obtained using mRNA purified kit (Oligotex-dT30 <super> mRNA Purification kit, purchased from Takara Shuzo Co.).

### [Example 8] Acquisition of cDNA fragments of anti-estrone monoclonal antibody heavy chain and light chain variable regions and determination of their base sequences as well as deduction of amino acid sequences of heavy chain and light chain variable regions

The synthesis of cDNAs was carried out using about 100 ng of mRNA each for light chain and heavy chain, with First Strand cDNA Synthesis Kit ReverTra Ace -α™ (purchased from Toyobo Co.). Then, amplification of each cDNA fragment was carried out by PCR reaction in Personal cycler (purchased from Biometra) (94°C 30 sec, 55°C 30 sec, 72°C 2 min; Repeat number: 30 cycles), using primers for heavy chain and primers for light chain shown in the following, which were devised from their N terminal amino acid sequences obtained in Example 1, and Pfu Turbo (purchased from Toyobo Co.) enzyme.
Primers for E1-420 heavy chain 
Forward 5'- CTATGCGGCCCAGCCGGCCATGATCCAGTTGGTGCAGTCTGGA-3'
(SEQ ID NO: 30)
 Back 5'- CGAGGAGACTGTGAGAGTGGT-3' (SEQ ID NO: 31) Primers for E1-420 light chain

Forward 5'- CTCAGTCTCCAGATGTTTTGATGACCCAAACT-3' (SEQ ID NO:
32)

Back 5'- CCGTTTCAGCTCCAGCTTGGTCCC-3' (SEQ ID NO: 33)

Thus, cDNA fragments for variable regions of heavy chain and light chain of anti-estradiol monoclonal antibody were obtained. Each fragment was purified from 2% agarose after electrophoresis using a micro spin column (purchased from Amersham Pharmacia Biotech Co.). Then, using dideoxy method, the base sequence of each variable region cDNA was determined, and the amino acid sequence was deduced therefrom. The base sequences (SEQ ID NOs: 5 and 7) and amino acid sequences (SEQ ID NOs: 6 and 8) were determined by DNA sequencer 310 (Applied Biosystems Japan), after the reaction in a Personal cycler (purchased from Biometra) with Big Dye Terminator Cycle Sequencing Ready Reaction Kit (purchased from Applied Biosystems Japan) using the above-mentioned PCR primers as sequencing primers.

Namely, SEQ ID NO: 5 depicts the base sequence of cDNA containing the heavy chain variable region of the antibody produced by mouse hybridoma cell line E1-420, SEQ ID NO: 6 depicts the amino acid sequence corresponding thereto.

SEQ ID NO: 7 depicts the base sequence of cDNA containing the light chain variable region of the antibody produced by mouse hybridoma cell line E1-420, SEQ ID NO: 8 depicts the amino acid sequence corresponding thereto.

### [Example 9] Determination of N terminal amino acid sequence of anti-ethynyl estradiol monoclonal antibody

The antibody protein purified from the culture supernatant of anti- ethynyl estradiol monoclonal antibody-producing mouse hybridoma cells (EE2-3 line or EE2-8-18 line) obtained in Reference Example 1 using Protein A column (PROSEP-A, purchased from Air Brown Co.) was separated and purified to heavy chain and light chain by SDS-polyacrylamide gel electrophoresis, and transferred onto PVDF membrane. After staining with Ponso-S, protein bands were excised. The excised proteins on the membrane were treated with Pfu Pyroglutamate Aminopeptidase (purchased from Takara Shuzo Co.) (2 mU) at 50°C for 17 hr, and N terminal amino acid sequences were determined with gas phase protein sequencer (Model 494 Applied Biosystems Japan). The following results were obtained.

EE2-3 heavy chain: VTLKESGPGILQPSQTLSLT (SEQ ID NO: 34)

EE2-3 light chain: DIVMSQSPSSLAVSVGEKVT (SEQ ID NO: 35)

EE2-8-18 heavy chain: VQVKESGPGLVAPSQSL (SEQ ID NO: 36)

EE2-8-18 light chain: DIQMTQSPASL?A?VG?TVT (SEQ ID NO: 37)

EE2-227 heavy chain: VTLKESGPGILQPSQTL (SEQ ID NO: 38)

EE2-227 light chain: DIVMSQSPSSLAVSVGE (SEQ ID NO: 39)

### [Example 10] Purification of anti-ethynyl estradiol monoclonal antibody mRNA

Anti-ethynyl estradiol monoclonal antibody producing mouse hybridoma cells (EE2-3 line or EE2-8-18 line) (1X10⁷ cells/ml) obtained in Reference Example 1 were suspended in RNA preparation solution (ISOGEN solution, purchased from Wako Pure Chemical Industries, Ltd.) (1 ml) and disrupted, and RNA was extracted from the cells according to the manual. The concentration of RNA was determined from absorbance at 260 nm. From the obtained RNA (about 100 µg), mRNA (about 1.5 µg) was obtained using mRNA purification kit (Oligotex-dT30 <super> mRNA Purification kit, purchased from Takara Shuzo Co.).

### [Example 11] Acquisition of cDNA fragments of anti-ethynyl estradiol monoclonal antibody heavy chain and light chain variable regions and determination of their base sequences as well as deduction of amino acid sequences of heavy chain and light chain variable regions

The synthesis of cDNAs was carried out using about 100 ng of mRNA each for light chain and heavy chain, with First Strand cDNA Synthesis Kit ReverTra Ace -α™ (purchased from Toyobo Co.). Then, amplification of each cDNA fragment was carried out by PCR reaction in Personal cycler (purchased from Biometra) (94°C 30 sec, 55°C 30 sec, 72°C 2 min; Repeat number: 30 cycles), using primers for heavy chain and primers for light chain shown in the following, which were devised from their N terminal amino acid sequences obtained in Example 1, and Pfu Turbo (purchased from Toyobo Co.) enzyme.
Primers for EE2-3 heavy chain <R:A,G> 
Forward 5'- CTATGCGGCCCAGCCGGCCCAGGTTACTCTRAAARARTC-3' (SEQ
ID NO: 40)
 Back 5'- CGCAGAGACAGTGACCAGAGT-3' (SEQ ID NO: 41) Primers for EE2-3 light chain 
Forward 5′- CTCAGTCTCCAGACATTGTGATGTCACAGTC-3′ (SEQ ID NO:
42)
 Back 5'- GCGGCCGCCTCATTCCTGTTGAAGCTCTTGAC-3' (SEQ ID NO: 43) Primers for EE2-8 - 18 heavy chain 
Forward 5′- CTATGCGGCCCAGCCGGCCATGGTGCAGGTGAAGGAGTCAGGA-3′
(SEQ ID NO: 44)
 Back 5'- CGAGGAGACGGTGACTGAGGT-3' (SEQ ID NO: 45) Primers for EE2-8 - 18 light chain <Y:C,T D:A,G,T> 
Forward 5'- CTCAGTCTCCAGAYATTCAGATGAYDCAGTC-3' (SEQ ID NO:
46)
 Back 5’- CCGTTTGATTTCCAGCTTGGTGCC-3’ (SEQ ID NO: 47) Primers for EE2-227 heavy chain <R:A,G> 
Forward 5′-CTATGCGGCCCAGCCGGCCCAGGTTACTCTRAAARARTC-3′ (SEQ ID
NO: 40)
 Back 5'-CGCAGAGACAGTGACCAGAGT-3'(SEQ ID NO: 41) Primers for EE2-8 - 227 light chain

Forward 5'-CTCAGTCTCCAGACATTGTGATGTCACAGTC-3' (SEQ ID NO: 48)

Back 5'-GCGGCCGCCTCATTCCTGTTGAAGCTCTTGAC-3' (SEQ ID NO: 49)

Thus, cDNA fragments for variable regions of heavy chain and light chain of anti-ethynyl estradiol monoclonal antibody were obtained. Each fragment was purified from 2% agarose after electrophoresis using a micro spin column (purchased from Amersham Pharmacia Biotech Co.). Then, using dideoxy method, the base sequence of each variable region cDNA was determined, and the amino acid sequence was deduced therefrom. The base sequences (SEQ ID NOs: 9,11,13 and 15) and amino acid sequences (SEQ ID NOs: 10,12,14 and 16) were determined by DNA sequencer 310 (Applied Biosystems Japan), after the reaction in a Personal cycler (purchased from Biometra) with Big Dye Terminator Cycle Sequencing Ready Reaction Kit (purchased from Applied Biosystems Japan) using the above-mentioned PCR primers as sequencing primers.

Namely, SEQ ID NO: 9 depicts the base sequence of cDNA containing the heavy chain variable region of the antibody produced by mouse hybridoma cell line EE2-3, SEQ ID NO: 10 depicts the amino acid sequence corresponding thereto. SEQ ID NO: 11 depicts the base sequence of cDNA containing the light chain variable region of the antibody produced by mouse hybridoma cell line EE2-3, SEQ ID NO: 12 depicts the amino acid sequence corresponding thereto. SEQ ID NO: 13 depicts the base sequence of cDNA containing the heavy chain variable region of the antibody produced by mouse hybridoma cell line EE2-8-18, SEQ ID NO: 14 depicts the amino acid sequence corresponding thereto.
SEQ ID NO: 15 depicts the base sequence of cDNA containing the light chain variable region of the antibody produced by mouse hybridoma cell line EE2-8-18, SEQ ID NO: 16 depicts the amino acid sequence corresponding thereto. SEQ ID NO: 17 depicts the base sequence of cDNA containing the heavy chain variable region of the antibody produced by mouse hybridoma cell line EE2-227, SEQ ID NO: 18 depicts the amino acid sequence corresponding thereto. SEQ ID NO: 19 depicts the base sequence of cDNA containing the light chain variable region of the antibody produced by mouse hybridoma cell line EE2-227, SEQ ID NO: 20 depicts the amino acid sequence corresponding thereto.

### [Example 12] Production of single chain variable region antibody containing anti-estrone monoclonal antibody heavy chain variable region and anti-estradiol monoclonal antibody light chain variable region (E1H-E2L scFv)

Using primers for anti-estrone monoclonal antibody heavy chain variable region (E1H) shown in the following, E1H cDNA was amplified by PCR. Then, using primers for anti- estradiol monoclonal antibody light chain variable region (E2L) shown in the following, E2L cDNA was amplified by PCR. Using the forward primer for anti-estrone monoclonal antibody heavy chain variable region (E1H) and the back primer for anti-estradiol monoclonal antibody light chain variable region (E2L), a single strand DNA (E1H-E2L cDNA) containing E1H cDNA and E2L cDNA was produced by PCR using the amplified E1H cDNA and E2L cDNA as templates. The PCR was carried out using Pfu Turbo (purchased from Toyobo Co.) enzyme (94°C 30 sec, 55°C 30 sec, 72°C 2 min; Repeat number: 30 cycles).
Primers for anti-estrone monoclonal antibody heavy chain variable region (E1H) 
forward 5′-CTATGCGGCCCAGCCGGCCATGATCCAGTTGGTGCAGTCTGGA -3′
(SEQ ID NO: 30)
 
back 5'-GAGCCACCTCCGCCTGAACCGCCTCCACCCGAGGAGACTGT -3' (SEQ ID
NO: 50)
 Primers for anti- estradiol monoclonal antibody light chain

forward 5'-CGGAGGTGGCTCTGGCGGTGGCGGATCGGATATCGTGATG -3' (SEQ
ID NO: 51)

back 5′-GCGCACCTGCGGCCGCCCGTTTTATTTC -3′ (SEQ ID NO: 52)

### [Example 13] Production of single chain variable region antibody containing anti-estradiol monoclonal antibody heavy chain variable region and anti-estrone monoclonal antibody light chain variable region (E2H-E1L scFv)

Using primers for anti-estradiol monoclonal antibody heavy chain variable region (E2H) shown in the following, E2H cDNA was amplified by PCR. Then, using primers for anti-estrone monoclonal antibody light chain variable region (E1L) shown in the following, E1L cDNA was amplified by PCR. Using the forward primer for anti-estradiol monoclonal antibody heavy chain variable region (E2H) and the back primer for anti-estrone monoclonal antibody light chain variable region (E1L), a single strand DNA (E2H-E1L cDNA) containing E2H cDNA and E1L cDNA was prepared by PCR using the amplified E2H cDNA and E1L cDNA as templates. The PCR was carried out using Pfu Turbo (purchased from Toyobo Co.) enzyme (94°C 30 sec, 55°C 30 sec, 72°C 2 mini Repeat number: 30 cycles).
Primers for anti-estradiol monoclonal antibody heavy chain variable region (E2H) 
forward 5′-CTATGCGGCCCAGCCGGCCATGGTBCAGCTGCAGCAGTCTGG-3′ (SEQ
ID NO: 23)
 
back 5'-GAGCCACCTCCGCCTGAACCGCCTCCACCCGAGGAGACTGT -3' (SEQ ID
NO: 53)
 Primers for anti-estrone monoclonal antibody light chain variable region (E1L)

forward 5'-CGGAGGTGGCTCTGGCGGTGGCGGATCGGATGTTTTGATG -3' (SEQ
ID NO: 54)

back 5'-GCGCACCTGCGGCCGCCCGTTTCAGCTC-3' (SEQ ID NO: 55)

### [Example 14] Production of single chain variable region antibodies (E1H-E2L scFv and E2H-E1L scFv)-expressing vectors

The single strand DNA (E1H-E2L cDNA) obtained in Example 13 was digested with restriction enzyme *Sfi*I and *Not*I. Then, to a solution (10 µl) containing E1H-E2L cDNA (41 ng) and pCANTAB5E (purchased from Amersham Pharmacia Biotech) predigested with SfiI and NotI (90 ng) were added the solution A (Reaction Buffer) (50 µl) and solution B (Enzyme Solution) (10 µl) of DNA ligation kit (TaKaRa), the mixture was reacted at 16°C for 2 hr. Then, the DNA sample was purified by alkali dissolution and ethanol precipitation, and dissolved in ultrapure water. Then, the obtained DNA sample (2 µl) was mixed with TG-1 competent cells (purchased from STRATAGENE), and electroporation was carried out with GENE Pulser II Electroportion System (BIO PAD) (200 Ω, 2.5 KV, Capa 25). And then, to this solution was added an appropriate amount of 2×YTG (1×YTG: in sterilized water (1,000 ml), Bacto-tryptone 8.5 g, Bacto-yeast extract 5 g, NaCl 2.5 g, 1% Glucose), the cells were transferred into a tube and cultured with shaking at 37°C for 1 hr, followed by plating onto a medium plate and overnight culture at 37°C. The next day, a colony on the plate was picked up and cultured in LB medium overnight, and a phagemid was purified by alkali dissolution and ethanol precipitation. Insertion of the objective insert DNA into the phagemid was confirmed by *Sfi*I and *Not*I treatment, thus a single chain variable region antibody (E1H-E2L scFv)-expressing vector pCANTABSE/E1H-E2L scFv was obtained. The E1H-E2L cDNA was also treated by the same method as in the case of E2H-E1L cDNA to yield a single chain variable region antibody (E2H-E1L scFv)-expressing vector pCANTAB5E/E2H-E1L scFv.

### [Example 15] Determination of binding ability of single chain variable region antibody (E1H-E2L scFv and E2H-E1L scFv)-expressing phages against estrone and estradiol

A colony containing E1H-E2L scFv and a colony containing a phage expressing E2H-E1L scFv were picked up from the medium plate, respectively, inoculated into LB medium containing ampicillin (concentration: 100 µg/ml) (1.6 ml) and cultured with shaking at 37°C overnight. The next day, the obtained culture solution (200 µl) was added to LB medium containing ampicillin (concentration 100 µg/ml) (20 ml), and cultured with shaking at 37°C for 2 hr. Two hours later, to this medium was added a helper phage M13KO7 solution (20 µl), and the mixture was standing-cultured at 28°C for 1 hr. One hour later, to this medium was added kanamycin (final concentration 50 µg/ml), and the mixture was cultured with shaking at 28°C overnight.

Then, the culture solution was transferred into a centrifugation tube and centrifuged (9,500 rpm, 4°C, 35 min). The supernatant (containing phage) was transferred to other centrifugation tube, and additionally centrifuged (11,000 rpm, 4°C, 30 min). The supernatant (containing phage) was transferred to other centrifugation tube, mixed with 20% PEG6000, 2.5 M NaCl (5 ml) and stood in ice for 1 hr. One hour later, the mixture was centrifuged (11,000 rpm, 4°C, 30 min) and the supernatant was removed. The mixture was lightly centrifuged and the supernatant was completely removed. The obtained precipitation was dissolved in PBS (300 µl) and stored at 4°C.

Then, antigens E1-6CMO:Ova (hereinafter abbreviated as E1-Ova; Ova stands for ovalbumin) and E2-3CME:Ova (hereinafter abbreviated as E2-Ova) were prepared in the same manner as in the above-mentioned method. And then, 10 µg/ml of Ova, E1-Ova or E2-Ova (100 µl) was added to each well, stood at 4°C overnight to be immobilized on the well. After the liquid was removed from the well, 3% skim milk (300 µl) was added to each well, the mixture was stood at 37°C for 1.5 hr. 1.5 hr later, the liquid was removed from the well, the well was washed with PBS twice. The solution containing a phage expressing E1H-E2L scFv was 2-fold diluted with PBS, and the 2-fold diluted phage solution (100 µl) was added to each well. The mixture was stood at room temperature for 2.5 hr.

After 2.5 hr, the liquid was removed from the well, the well was washed with 0.05% Tween/PBS (twice) and PBS (once). Then, a secondary antibody solution (2500-fold diluted HRP/Anti-M13 (purchased from Amersham Pharmacia Biotech) with 0.1% skim milk/PBS) (100 µl) was added to each well, the mixture was stood at room temperature for 1.5 hr. After 1.5 hr, the well was washed with 0.05% Tween/PBS (twice) and PBS (once). TMB solution (purchased from Wako Pure Chemical Industries, Ltd.) was added, and the mixture was reacted with shaking for 30 min. Thirty minutes later, 1 N HCl was added to stop reaction. Then, the absorbance at 450 nm was measured. The results are shown in Fig. 2.

As a result, the absorbance in the well on which antigen E1-Ova or E2-Ova was immobilized was higher than that in the well on which Ova was immobilized.

From the above, it has been revealed that a phage expressing E1H-E2L scFv and a phage expressing E2H-E1L scFv binds to both estrone and estradiol.

### [Example 16] Change of binding ability for estrone and estradiol with dilution of phage expressing a single chain variable region antibody (E2H-E1L scFv)

In the same manner as in Example 15, the binding ability of a phage expressing E2H-E1L scFv for estrone and estradiol was assayed, provided that 16-fold diluted, 64-fold diluted, and 256-fold diluted phage solutions were used in this instance, whereas 2-fold diluted phage solution was used in Example 15. The results are shown in Fig. 3.

As a result, a phage expressing E2H-E1L scFv reacted with antigens E1-Ova and E2-Ova in a concentration-dependent manner, and in all dilutions absorbance in the cases of E1-Ova and E2-Ova was higher than that in the case of Ova. Furthermore, this antibody strongly reacted with E2-Ova, also well reacted with E1-Ova.

From the above, it has been revealed that E2H-E1L scFv is expressed on the phage and reacts with both antigens.

### [Example 17] Comparison of binding ability for estrone and estradiol between a monoclonal antibody and a phage expressing ScFv

Then, binding ability for an antigen was compared between a phage expressing a scFv wherein the heavy chain and light chain variable regions of anti-estrone monoclonal antibody E1-420 were ligated (produced in the same manner as in Example 5) and the monoclonal antibody E1-420, or between a scFv wherein the heavy chain and light chain variable regions of anti-estradiol monoclonal antibody E2-733 were ligated (produced in Example 5) and the anti-estradiol monoclonal antibody E2-733. The results are shown in Fig. 4.

As a result, both scFv-expressing phages showed the same level of binding ability for antigen as the original monoclonal antibodies. Meanwhile, the reason that the gradient of the dilution curve of scFv-expressing phage is gentle is assumed that the reaction time is too short to make the reaction come to equilibrium. Particularly, because of high molecular weight of phage, a remarkable difference would appear in E1 having a weaker binding ability.

These results show that a phage antibody useful for the detection of female sex hormones could be obtained.

### Industrial Applicability

According to the present invention, a gene encoding a heavy chain or light chain of an anti-female sex hormone antibody is provided and the amino acid and base sequence thereof is clarified. It has become possible to genetically modify genes provided by the present invention, especially the genes encoding the variable regions of heavy chains and light chains; it has become possible to obtain proteins capable of binding to female sex hormones, and having preferred characteristics for measuring, quantifying and concentrating female sex hormones in large amounts, by expressing the thus-modified genes in host cells. Using a transformant microbial cell etc. harboring this modified antibody gene, it is possible to efficiently produce a recombinant protein. It is thereby possible to prepare an enzyme immunoassay kit and antibody affinity column of better performance at lower costs.

Also, the present invention has made it possible to prepare a phage antibody that expresses a single-chain antibody having the variable region of a heavy chain or light chain of an anti-female sex hormone antibody. Hence, it has become possible to create an antibody suitable for the fractionation or simultaneous analysis of various estrogens by means of a combination of these variable region fragments.

This application is based on a patent application No. 55669/2002 filed on March 1, 2002 in Japan, the contents of which are hereby incorporated by reference.

SEQUENCE LISTING

<110>  TAKEDA CHEMICAL INDUSTRIES, LTD.
<120>  A protin binding to female hormones and a production thereof
<130>  09534

<150>  JP 2002-055669
<151>  2002-03-01

<160>  59

<210>  1
<211>  351
<212>  DNA
<213>  Mouse

<400>  1
gtgcagctgc agcagtctgg ggctgaactg gctagacctg gggcttcagt gaaattgtcc      60

tgcaaggctt ctgactacac ctttactaac tactggatgc agtggataaa acagaggcct     120

ggacagggtc tggaatggat tgggactatt tctcttggaa atggtgatac cagatacact     180

cagaagttca aggacaaggc cacattgact gcagataaat cctccagtac agtctacatg     240

caactcacca acttggcatc tgaggattct gcggtctatt actgttcaat ctattccttc     300

gttaggggcc ccggaggttg gggccaaggc accactctca cagtctcctc g              351

<210>  2
<211>  117
<212>  PRT
<213>  Mouse

<400>  2
Val Gln Leu Gln Gln Ser Gly Ala Glu Leu Ala Arg Pro Gly Ala Ser
1               5                   10                  15

Val Lys Leu Ser Cys Lys Ala Ser Asp Tyr Thr Phe Thr Asn Tyr Trp
            20                  25                  30

Met Gln Trp Ile Lys Gln Arg Pro Gly Gln Gly Leu Glu Trp Ile Gly
        35                  40                  45

Thr Ile Ser Leu Gly Asn Gly Asp Thr Arg Tyr Thr Gln Lys Phe Lys
    50                  55                  60

Asp Lys Ala Thr Leu Thr Ala Asp Lys Ser Ser Ser Thr Val Tyr Met
65                  70                  75                  80

Gln Leu Thr Asn Leu Ala Ser Glu Asp Ser Ala Val Tyr Tyr Cys Ser
                85                  90                  95

Ile Tyr Ser Phe Val Arg Gly Pro Gly Gly Trp Gly Gln Gly Thr Thr
            100                 105                 110

Leu Thr Val Ser Ser
        115     117

<210>  3
<211>  324
<212>  DNA
<213>  Mouse

<400> 3
gatatcgtga tgactcagtc tcccacattc ttgtctacat cattaggaga caaaatcacc 60

atcacctgca aggccagtca ggatgtgggt cccgctatag cctggtatca acagacacca 120

ggacaatctc ctagactact gatttattgg acatccaccc ggcacactgg agtccctgat 180

cgcttcacag gcagtggatc tgggacagat ttcactctct ccattggcga tgttcagtct 240

gaagacttga cagattattt ctgtcaccaa tatcgcagct atccatacac attcgggggg 300

gggaccaagc tggaaataaa acgg 324

<210> 4
<211> 108
<212> PRT
<213> Mouse

<400> 4
Asp Ile Val Met Thr Gln Ser Pro Thr Phe Leu Ser Thr Ser Leu Gly
1               5                   10                  15

Asp Lys Ile Thr Ile Thr Cys Lys Ala Ser Gln Asp Val Gly Pro Ala
            20                  25                  30

Ile Ala Trp Tyr Gln Gln Thr Pro Gly Gln Ser Pro Arg Leu Leu Ile
        35                  40                  45

Tyr Trp Thr Ser Thr Arg His Thr Gly Val Pro Asp Arg Phe Thr Gly
    50                  55                  60

Ser Gly Ser Gly Thr Asp Phe Thr Leu Ser Ile Gly Asp Val Gln Ser
65                  70                  75                  80

Glu Asp Leu Thr Asp Tyr Phe Cys His Gln Tyr Arg Ser Tyr Pro Tyr
                85                  90                  95

Thr Phe Gly Gly Gly Thr Lys Leu Glu Ile Lys Arg
100 105 108

<210> 5
<211> 354
<212> DNA
<213> Mouse

<400> 5
atccagttgg tgcagtctgg acctgaactg aagaagcctg gagagacagt caagatctcc 60

tgcaaggctt ctgggtttac cttcagaaac tatggaatga actgggtgag gcagtctcca 120

ggagagggtt taaagtggat gggctggata aacacctaca ctggagagcc aacatatcct 180

gatgccttca agggacggtt tgccatctct ctggaaacct ctgccagcac tgcctatttg 240

cagatcaaca gcctcaaaaa tgaggacacg gctacatatt tctgtgcaag aggggactat 300

aggtaccgtt cctttgacta ctggggccaa ggcaccactc tcacagtctc ctcg 354

<210> 6
<211> 118
<212> PRT
<213> Mouse

<400> 6
Ile Gln Leu Val Gln Ser Gly Pro Glu Leu Lys Lys Pro Gly Glu Thr
1               5                   10                  15

Val Lys Ile Ser Cys Lys Ala Ser Gly Phe Thr Phe Arg Asn Tyr Gly
            20                  25                  30

Met Asn Trp Val Arg Gln Ser Pro Gly Glu Gly Leu Lys Trp Met Gly
        35                  40                  45

Trp Ile Asn Thr Tyr Thr Gly Glu Pro Thr Tyr Pro Asp Ala Phe Lys
    50                  55                  60

Gly Arg Phe Ala Ile Ser Leu Glu Thr Ser Ala Ser Thr Ala Tyr Leu
65                  70                  75                  80

Gln Ile Asn Ser Leu Lys Asn Glu Asp Thr Ala Thr Tyr Phe Cys Ala
                85                  90                  95

Arg Gly Asp Tyr Arg Tyr Arg Ser Phe Asp Tyr Trp Gly Gln Gly Thr
            100                 105                 110

Thr Leu Thr Val Ser Ser
        115         118

<210>  7
<211>  339
<212>  DNA
<213>  Mouse

<400>  7
gatgttttga tgacccaaac tccactcact ttgtcggtta ccattggaca accagcctcc      60

atctcttgca agtcaagtca gagcctctta aatagtgatg gaaagacata tttgcattgg     120

ttgttacaga ggccaggcca gtctccaaag cgcctaatct atctggtgtc taaactggac     180

tctggagtcc ctgacaggtt ctctggcagt ggatcaggga cagatttcac actgaaaatc     240

agcagagtgg aggctgagga tttgggagtt tattattgct ggcaagttac acattttcct     300

ctcacgttcg gtgctgggac caagctggag ctgaaacgg                            339

<210>  8
<211>  113
<212>  PRT
<213>  Mouse

<400>  8
Asp Val Leu Met Thr Gln Thr Pro Leu Thr Leu Ser Val Thr Ile Gly
1               5                   10                  15

Gln Pro Ala Ser Ile Ser Cys Lys Ser Ser Gln Ser Leu Leu Asn Ser
            20                  25                  30

Asp Gly Lys Thr Tyr Leu His Trp Leu Leu Gln Arg Pro Gly Gln Ser
        35                  40                  45

Pro Lys Arg Leu Ile Tyr Leu Val Ser Lys Leu Asp Ser Gly Val Pro
    50                  55                  60

Asp Arg Phe Ser Gly Ser Gly Ser Gly Thr Asp Phe Thr Leu Lys Ile

65                  70                  75                  80

Ser Arg Val Glu Ala Glu Asp Leu Gly Val Tyr Tyr Cys Trp Gln Val
                85                  90                  95

Thr His Phe Pro Leu Thr Phe Gly Ala Gly Thr Lys Leu Glu Leu Lys
            100                 105                 110

Arg
113

<210>  9
<211>  354
<212>  DNA
<213>  Mouse

<400>  9
gttactctga aaxagtctgg ccctgggata ttgcagccct cccagaccct cagtctgact      60

tgttctttct ctgggttttc actgagcact tctgatctgg gtgtgagttg gattcgtcag     120

acttcaggaa agggtctgga gtggctggca cacatttact ggaatgatga caaatattat     180

aatccatccc tgaagagccg gctcacaatc tccaaggata cctccggaaa cctggtattc     240

ctcaggatca ccagtgtgga cactgacgat tctgccacat actattgtgc tcgaagtcct     300

ctctacggta gtcttactta ctggggccaa gggactctgg tcactgtctc tgcg           354

<210>  10
<211>  118
<212>  PRT
<213>  Mouse

<400>  10
Val Thr Leu Lys Glu Ser Gly Pro Gly Ile Leu Gln Pro Ser Gln Thr
1               5                   10                  15

Leu Ser Leu Thr Cys Ser Phe Ser Gly Phe Ser Leu Ser Thr Ser Asp
            20                  25                  30

Leu Gly Val Ser Trp Ile Arg Gln Thr Ser Gly Lys Gly Leu Glu Trp
        35                  40                  45

Leu Ala His Ile Tyr Trp Asn Asp Asp Lys Tyr Tyr Asn Pro Ser Leu
    50                  55                  60

Lys Ser Arg Leu Thr Ile Ser Lys Asp Thr Ser Gly Asn Leu Val Phe
65                  70                  75                  80

Leu Arg Ile Thr Ser Val Asp Thr Asp Asp Ser Ala Thr Tyr Tyr Cys
                85                  90                  95

Ala Arg Ser Pro Leu Tyr Gly Ser Leu Thr Tyr Trp Gly Gln Gly Thr
            100                 105                 110

Leu Val Thr Val Ser Ala
        115         118

<210>  11
<211>  654
<212>  DNA
<213>  Mouse

<400> 11
gacattgtga tgtcacagtc tccatcctcc ctggctgtgt cagtaggaga gaaggtcact 60

ttgaactgca aatccagtca gagtctgctc aacagtagaa cccgaaagaa ctacttggct 120

tggtaccagc agaaaccagg acagtctcct aaactgctga tctcttgggc atccactagg 180

gaatctgggg tccctgatcg tttcacaggc agtggatctg ggacagattt cactctcacc 240

atcagcagtg tgcaggctga agacctggca gtttattact gcaatcaatt ttattatcta 300

ctcacgttcg gtgctgggac caagctggag ctgaaacggg ctgatgctgc accaactgta 360

tccatcttcc caccatccag tgagcagtta acatctggag gtgcctcagt cgtgtgcttc 420

ttgaacaact tctaccccaa agacatcaat gtcaagtgga agattgatgg cagtgaacga 480

caaaatggcg tcctgaacag ttggactgat caggacagca aagacagcac ctacagcatg 540

agcagcaccc tcacgttgac caaggacgag tatgaacgac ataacagcta tacctgtgag 600

gccactcaca agacatcaac ttcacccatt gtcaagagct tcaacaggaa tgag 654

<210> 12
<211> 218
<212> PRT
<213> Mouse

<400> 12
Asp Ile Val Met Ser Gln Ser Pro Ser Ser Leu Ala Val Ser Val Gly
1               5                   10                  15

Glu Lys Val Thr Leu Asn Cys Lys Ser Ser Gln Ser Leu Leu Asn Ser
            20                  25                  30

Arg Thr Arg Lys Asn Tyr Leu Ala Trp Tyr Gln Gln Lys Pro Gly Gln
        35                  40                  45

Ser Pro Lys Leu Leu Ile Ser Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Asn Gln
                85                  90                  95

Phe Tyr Tyr Leu Leu Thr Phe Gly Ala Gly Thr Lys Leu Glu Leu Lys
            100                 105                 110

Arg Ala Asp Ala Ala Pro Thr Val Ser Ile Phe Pro Pro Ser Ser Glu
        115                 120                 125

Gln Leu Thr Ser Gly Gly Ala Ser Val Val Cys Phe Leu Asn Asn Phe
    130                 135                 140

Tyr Pro Lys Asp Ile Asn Val Lys Trp Lys Ile Asp Gly Ser Glu Arg
145                 150                 155                 160

Gln Asn Gly Val Leu Asn Ser Trp Thr Asp Gln Asp Ser Lys Asp Ser
                165                 170                 175

Thr Tyr Ser Met Ser Ser Thr Leu Thr Leu Thr Lys Asp Glu Tyr Glu
            180                 185                 190

Arg His Asn Ser Tyr Thr Cys Glu Ala Thr His Lys Thr Ser Thr Ser
        195                 200                 205

Pro Ile Val Lys Ser Phe Asn Arg Asn Glu
    210                 215         218

<210>  13
<211>  355
<212>  DNA
<213>  Mouse

<400>  13
gtgcaggtga aggagtcagg acctggcctg gtggcgccct cacagagcct gtccatcagt      60

tgcactgtct ctgggtttcc attaaccagt tatggtgtac agtgggttcg ccagcctcca     120

ggaaagggtc tggagtggct gggagtaata tggcctggtg gaagcacaaa ttataattcg     180

gctctcatgt ccagactgag catcagcaaa gacaactcca agagccaagt tttcttaaaa     240

atgaacagtc tgcaaactga tgacacagcc atgtactact gtgccggatg gttccaaacg     300

ggaccgaggc tatttgctta ctggggccaa gggactctgg tcactgtctc tgcga          355

<210>  14
<211>  118
<212>  PRT
<213>  Mouse

<400>  14
Val Gln Val Lys Glu Ser Gly Pro Gly Leu Val Ala Pro Ser Gln Ser
1               5                   10                  15

Leu Ser Ile Ser Cys Thr Val Ser Gly Phe Pro Leu Thr Ser Tyr Gly
            20                  25                  30

Val Gln Trp Val Arg Gln Pro Pro Gly Lys Gly Leu Glu Trp Leu Gly
        35                  40                  45

Val Ile Trp Pro Gly Gly Ser Thr Asn Tyr Asn Ser Ala Leu Met Ser
    50                  55                  60

Arg Leu Ser Ile Ser Lys Asp Asn Ser Lys Ser Gln Val Phe Leu Lys
65                  70                  75                  80

Met Asn Ser Leu Gln Thr Asp Asp Thr Ala Met Tyr Tyr Cys Ala Gly
                85                  90                  95

Trp Phe Gln Thr Gly Pro Arg Leu Phe Ala Tyr Trp Gly Gln Gly Thr
            100                 105                 110

Leu Val Thr Val Ser Ala
        115         118

<210>  15
<211>  321
<212>  DNA
<213>  Mouse

<400>  15

xxxxxxxxxx xxxxxxxxxx xccagcctcc ctatctgcat ctgtgggaga aactgtcacc 60

atcacatgtc gagcaagtga gaatattcac agttatttag catggtatca gcagaaacag 120

ggaaaatctc ctcaggtcct ggtctataat gcaaaaacct taggagaagg tgtgtcatca 180

aggttcagtg gcagtggatc aggcacacag ttttctctga agatcaacag cctgcagcct 240

gaagattttg ggacttatta ctgtcaaaat cattatggta gtccgtggac gttcggtgga 300

ggcaccaagc tggaatcaaa a 321

<210> 16
<211> 107
<212> PRT
<213> Mouse

<400> 16
Asp Ile Gln Met Thr Gln Ser Pro Ala Ser Leu Ser Ala Ser Val Gly
1               5                   10                  15

Glu Thr Val Thr Ile Thr Cys Arg Ala Ser Glu Asn Ile His Ser Tyr
            20                  25                  30

Leu Ala Trp Tyr Gln Gln Lys Gln Gly Lys Ser Pro Gln Val Leu Val
        35                  40                  45

Tyr Asn Ala Lys Thr Leu Gly Glu Gly Val Ser Ser Arg Phe Ser Gly
    50                  55                  60

Ser Gly Ser Gly Thr Gln Phe Ser Leu Lys Ile Asn Ser Leu Gln Pro
65                  70                  75                  80

Glu Asp Phe Gly Thr Tyr Tyr Cys Gln Asn His Tyr Gly Ser Pro Trp
                85                  90                  95

Thr Phe Gly Gly Gly Thr Lys Leu Glu Ser Lys
100 105 107

<210> 17
<211> 355
<212> DNA
<213> Mouse

<400> 17
gttactctga aagagtctgg ccctgggata ttgcagccct cccagaccct cagtctgact 60

tgttctttct ctgggttttc actgagcact tctgatctgg gtgtgagttg gattcgtcag 120

cgttcaggaa agggtctgga gtggctggca cacatttact ggaatgatga caaatactat 180

aatccatacc tgaggagccg gctcacaatc tccaaggata ccgccggaaa cctggtattc 240

ctcaggatca ccagtgtgga cactgacgat tctgccacat actattgtgc tcgaagccct 300

ctctacggta gtctttctta ctggggccaa gggactctgg tcactgtctc tgcga 355

<210> 18
<211> 118
<212> PRT
<213> Mouse

<400> 18

Val Thr Leu Lys Glu Ser Gly Pro Gly Ile Leu Gln Pro Ser Gln Thr
1               5                   10                  15

Leu Ser Leu Thr Cys Ser Phe Ser Gly Phe Ser Leu Ser Thr Ser Asp
            20                  25                  30

Leu Gly Val Ser Trp Ile Arg Gln Arg Ser Gly Lys Gly Leu Glu Trp
        35                  40                  45

Leu Ala His Ile Tyr Trp Asn Asp Asp Lys Tyr Tyr Asn Pro Tyr Leu
    50                  55                  60

Arg Ser Arg Leu Thr Ile Ser Lys Asp Thr Ala Gly Asn Leu Val Phe
65                  70                  75                  80

Leu Arg Ile Thr Ser Val Asp Thr Asp Asp Ser Ala Thr Tyr Tyr Cys
                85                  90                  95

Ala Arg Ser Pro Leu Tyr Gly Ser Leu Ser Tyr Trp Gly Gln Gly Thr
            100                 105                 110

Leu Val Thr Val Ser Ala
        115

<210>  19
<211>  626
<212>  DNA
<213>  Mouse

<400>  19
gacattgtga tgtcacagtc tccatcctcc ctggctgtgt cagtaggaga gaaggtcact      60

atgaactgca aagccagtca gagtctgctc aacagtagaa cccgaaagaa ccacttggct     120

tggtaccagc agaaaccagg acagtctcca acactgttga tctcttgggc atccactagg     180

gaatctgggg tccctgatcg tttcacaggc agtggatctg ggacagattt cactctcacc     240

atcagcagtg tgcaggctga agacctggca gtttattact gcaatcaatt tttttatctg     300

ctcacgttcg gtgctgggac caagctggag ctgaaacggg ctgatgctgc accaactgta     360

tccatcttcc caccatccag tgagcagtta acatctggag gtgcctcagt cgtgtgcttc     420

ttgaacaact tctaccccaa agacatcaat gtcaagtgga agattgatgg cagtgaacga     480

caaaatggcg tcctgaacag ttggactgat caggacagca aagacagcac ctacagcatg     540

agcagcaccc tcacgttgac caaggacgag tatgaacgac ataacagcta tacctgtgag     600

gccactcaca agacatcaac ttcacc                                          626

<210>  20
<211>  208
<212>  PRT
<213>  Mouse

<400>  20
Asp Ile Val Met Ser Gln Ser Pro Ser Ser Leu Ala Val Ser Val Gly
1               5                   10                  15

Glu Lys Val Thr Met Asn Cys Lys Ala Ser Gln Ser Leu Leu Asn Ser
            20                  25                  30

Arg Thr Arg Lys Asn His Leu Ala Trp Tyr Gln Gln Lys Pro Gly Gln
        35                  40                  45

Ser Pro Thr Leu Leu Ile Ser Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Asn Gln
                85                  90                  95

Phe Phe Tyr Leu Leu Thr Phe Gly Ala Gly Thr Lys Leu Glu Leu Lys
            100                 105                 110

Arg Ala Asp Ala Ala Pro Thr Val Ser Ile Phe Pro Pro Ser Ser Glu
        115                 120                 125

Gln Leu Thr Ser Gly Gly Ala Ser Val Val Cys Phe Leu Asn Asn Phe
    130                 135                 140

Tyr Pro Lys Asp Ile Asn Val Lys Trp Lys Ile Asp Gly Ser Glu Arg
145                 150                 155                 160

Gln Asn Gly Val Leu Asn Ser Trp Thr Asp Gln Asp Ser Lys Asp Ser
                165                 170                 175

Thr Tyr Ser Met Ser Ser Thr Leu Thr Leu Thr Lys Asp Glu Tyr Glu
            180                 185                 190

Arg His Asn Ser Tyr Thr Cys Glu Ala Thr His Lys Thr Ser Thr Ser
        195                 200                 205

<210>  21
<211>  20
<212>  PRT
<213>  Mouse

<400>  21
Val Gln Leu Gln Gln Ser Gly Ala Glu Leu Ala Arg Pro Gly Ala Ser
1               5                   10                  15

Val Lys Leu Ser
            20

<210>  22
<211>  20
<212>  PRT
<213>  Mouse

<400>  22
Asp Ile Val Met Thr Gln Ser Pro Thr Phe Leu Ser Thr Ser Leu Gly
1               5                   10                  15

Asp Lys Ile Thr
            20

<210>  23
<211>  42
<212>  DNA
<213>  Artificial

<220>
<223>  Primer

<400>  23
ctatgcggcc cagccggcca tggtbcagct gcagcagtct gg                    42

<210>  24
<211>  22
<212>  DNA
<213>  Artificial

<220>
<223>  Primer

<400>  24
accactctca cagtctcctc gg                                          22

<210>  25
<211>  32
<212>  DNA
<213>  Artificial

<220>
<223>  Primer

<400>  25
ctcagtctcc agayatcgtg atgacycagt ct                               32

<210>  26
<211>  25
<212>  DNA
<213>  Artificial

<220>
<223>  Primer

<400>  26
gggaccaagc tggaaataaa acgga                                       25

<210>  27
<211>  25
<212>  PRT
<213>  Artificial

<220>
<223>  Linker

<400>  27
Gly Gly Gly Gly Ser
1               5

<210>  28
<211>  20
<212>  PRT
<213>  Mouse

<400>  28
Ile Gln Leu Val Glu Ser Gly Pro Glu Leu Lys Lys Pro Gly Glu Thr
1               5                   10                  15

Val Lys Ile Ser
            20

<210> 29
<211> 19
<212> PRT
<213> Mouse

<400> 29
Asp Val Leu Met Thr Gln Thr Pro Leu Thr Leu Ser Val Thr Ile Gly
1               5                   10                  15

Gln Pro Ala


<210> 30
<211> 43
<212> DNA
<213> Artificial

<220>
<223> Primer

<400> 30
ctatgcggcc cagccggcca tgatccagtt ggtgcagtct gga                43

<210> 31
<211> 21
<212> DNA
<213> Artificial

<220>
<223> Primer

<400> 31
cgaggagact gtgagagtgg t                                        21

<210> 32
<211> 32
<212> DNA
<213> Artificial

<220>
<223> Primer

<400> 32
ctcagtctcc agatgttttg atgacccaaa ct                            32

<210> 33
<211> 24
<212> DNA
<213> Artificial

<220>
<223> Primer

<400> 33
ccgtttcagc tccagcttgg tccc                                     24

<210> 34
<211> 20
<212> PRT
<213> Mouse

<400> 34
Val Thr Leu Lys Glu Ser Gly Pro Gly Ile Leu Gln Pro Ser Gln Thr

1               5                   10                  15

Leu Ser Leu Thr
            20

<210>  35
<211>  20
<212>  PRT
<213>  Mouse

<400>  35
Asp Ile Val Met Ser Gln Ser Pro Ser Ser Leu Ala Val Ser Val Gly
1               5                   10                  15

Glu Lys Val Thr
            20

<210>  36
<211>  18
<212>  PRT
<213>  Mouse

<400>  36
Val Gln Val Lys Glu Ser Gly Pro Gly Leu Val Ala Pro Ser Gln Ser
1               5                   10                  15

Leu

<210>  37
<211>  17
<212>  PRT
<213>  Mouse

<400>  37
Asp Ile Gln Met Thr Gln Ser Pro Ala Ser Leu Xaa Ala Xaa Val Gly
1               5                  10                  15

Xaa Thr Val Thr
            20

<210>  38
<211>  17
<212>  PRT
<213>  Mouse

<400>  38
Val Thr Leu Lys Glu Ser Gly Pro Gly Ile Leu Gln Pro Ser Gln Thr
1               5                   10                  15

Leu

<210>  39
<211>  17
<212>  PRT
<213>  Mouse

<400>  39
Asp Ile Val Met Ser Gln Ser Pro Ser Ser Leu Ala Val Ser Val Gly
1               5                   10                  15

Glu

<210> 40
<211> 39
<212> DNA
<213> Artificial

<220>
<223> Primer

<400> 40
ctatgcggcc cagccggccc aggttactct raaarartc 39

<210> 41
<211> 21
<212> DNA
<213> Artificial

<220>
<223> Primer

<400> 41
cgcagagaca gtgaccagag t 21

<210> 42
<211> 31
<212> DNA
<213> Artificial

<220>
<223> Primer

<400> 42
ctcagtctcc agacattgtg atgtcacagt c 31

<211> 32
<212> DNA
<213> Artificial

<220>
<223> Primer

<400> 43
gcggccgcct cattcctgtt gaagctcttg ac 32

<210> 44
<211> 43
<212> DNA
<213> Artificial

<220>
<223> Primer

<400> 44
ctatgcggcc cagccggcca tggtgcaggt gaaggagtca gga 43

<210> 45
<211> 21
<212> DNA
<213> Artificial

<220>
<223> Primer

<400> 45

cgaggagacg gtgactgagg t                                                21

<210>  46
<211>  31
<212>  DNA
<213>  Artificial

<220>
<223>  Primer

<400>  46
ctcagtctcc agayattcag atgaydcagt c                                     31

<210>  47
<211>  24
<212>  DNA
<213>  Artificial

<220>
<223>  Primer

<400>  47
ccgtttgatt tccagcttgg tgcc                                             24

<210>  48
<211>  31
<212>  DNA
<213>  Artificial

<220>
<223>  Primer

<400>  48
ctcagtctcc agacattgtg atgtcacagt c                                     31

<210>  49
<211>  32
<212>  DNA
<213>  Artificial

<220>
<223>  Primer

<400>  49
gcggccgcct cattcctgtt gaagctcttg ac                                    32

<210>  50
<211>  41
<212>  DNA
<213>  Artificial

<220>
<223>  Primer

<400>  50
gagccacctc cgcctgaacc gcctccaccc gaggagactg t                          41

<210>  51
<211>  40
<212>  DNA
<213>  Artificial

<220>

<223>  Primer

<400>  51
cggaggtggc tctggcggtg gcggatcgga tatcgtgatg                              40

<210>  52
<211>  28
<212>  DNA
<213>  Artificial

<220>
<223>  Primer

<400>  52
gcgcacctgc ggccgcccgt tttatttc                                           28

<210>  53
<211>  41
<212>  DNA
<213>  Artificial

<220>
<223>  Primer

<400>  53
gagccacctc cgcctgaacc gcctccaccc gaggagactg t                            41

<210>  54
<211>  40
<212>  DNA
<213>  Artificial

<220>
<223>  Primer

<400>  54
cggaggtggc tctggcggtg gcggatcgga tgttttgatg                              40

<210>  55
<211>  40
<212>  DNA
<213>  Artificial

<220>
<223>  Primer

<400>  55
cggaggtggc tctggcggtg gcggatcgga tgttttgatg                              40

<210>  56
<211>  14
<212>  PRT
<213>  Artificial

<220>
<223>  Linker

<400>  56
Gly Ser Thr Ser Gly Ser Gly Lys Ser Ser Glu Gly Lys Gly
1               5                   10

<210>  57
<211>  18

<212>  PRT
<213>  Artificial

<220>
<223>  Linker

<400>  57
Gly Ser Thr Ser Gly Ser Gly Lys Ser Ser Glu Gly Ser Gly Ser Thr
1               5                   10                  15

Lys Gly

<210>  58
<211>  12
<212>  PRT
<213>  Artificial

<220>
<223>  Linker

<400>  58
Gly Ser Thr Ser Gly Lys Pro Ser Glu Gly Lys Gly
1               5                   10

<210>  59
<211>  18
<212>  PRT
<213>  Artificial

<220>
<223>  Linker

<400>  59
Gly Ser Thr Ser Gly Ser Gly Lys Pro Gly Ser Gly Glu Gly Ser Thr
1               5                   10                  15

Lys Gly

## Claims

1. A protein of the following [a] or [b], or a salt thereof:
[a] a protein having an amino acid sequence shown by SEQ ID No: 2, an amino acid sequence shown by SEQ ID No: 6, an amino acid sequence shown by SEQ ID No: 10, an amino acid sequence shown by SEQ ID No: 14, an amino acid sequence shown by SEQ ID No: 18 or an amino acid sequence substantially the same as these;
[b] a protein having an amino acid sequence shown by SEQ ID No: 4, an amino acid sequence shown by SEQ ID No: 8, an amino acid sequence shown by SEQ ID No: 12, an amino acid sequence shown by SEQ ID No: 16, an amino acid sequence shown by SEQ ID No: 20 or an amino acid sequence substantially the same as these.

2. A protein of any of the following [a¹]-[a³] and [b¹]-[b³], or a salt thereof:
[a¹] a protein consisting of an amino acid sequence of any of the following (i)-(v), wherein a particular region is selected from the group consisting of a complementarity determining region 1, a complementarity determining region 2, a complementarity determining region 3, a framework region 1, a framework region 2, a framework region 3 and a framework region 4,
(i) an amino acid sequence wherein an amino acid sequence corresponding to not less than one particular region of the amino acid sequence shown by SEQ ID No: 2 has been exchanged for an amino acid sequence corresponding to the same kind of not less than one particular region contained in an amino acid sequence shown by SEQ ID No: 6, an amino acid sequence shown by SEQ ID No: 10, an amino acid sequence shown by SEQ ID No: 14 or an amino acid sequence shown by SEQ ID No: 18,
(ii) an amino acid sequence wherein an amino acid sequence corresponding to not less than one particular region of the amino acid sequence shown by SEQ ID No: 6 has been exchanged for an amino acid sequence corresponding to the same kind of not less than one particular region contained in an amino acid sequence shown by SEQ ID No: 2, an amino acid sequence shown by SEQ ID No: 10, an amino acid sequence shown by SEQ ID No: 14 or an amino acid sequence shown by SEQ ID No: 18,
(iii) an amino acid sequence wherein an amino acid sequence corresponding to not less than one particular region of the amino acid sequence shown by SEQ ID No: 10 has been exchanged for an amino acid sequence corresponding to the same kind of not less than one particular region contained in an amino acid sequence shown by SEQ ID No: 2, an amino acid sequence shown by SEQ ID No: 6, an amino acid sequence shown by SEQ ID No: 14 or an amino acid sequence shown by SEQ ID No: 18,
(iv) an amino acid sequence wherein an amino acid sequence corresponding to not less than one particular region of the amino acid sequence shown by SEQ ID No: 14 has been exchanged for an amino acid sequence corresponding to the same kind of not less than one particular region contained in an amino acid sequence shown by SEQ ID No: 2, an amino acid sequence shown by SEQ ID No: 6, an amino acid sequence shown by SEQ ID No: 10 or an amino acid sequence shown by SEQ ID No: 18, and
(v) an amino acid sequence wherein an amino acid sequence corresponding to not less than one particular region of the amino acid sequence shown by SEQ ID No: 18 has been exchanged for an amino acid sequence corresponding to the same kind of not less than one particular region contained in an amino acid sequence shown by SEQ ID No: 2, an amino acid sequence shown by SEQ ID No: 6, an amino acid sequence shown by SEQ ID No: 10 or an amino acid sequence shown by SEQ ID No: 14;
[b¹] a protein consisting of an amino acid sequence of any of the following (i) - (v), wherein a particular region is selected from the group consisting of a complementarity determining region 1, a complementarity determining region 2, a complementarity determining region 3, a framework region 1, a framework region 2, a framework region 3 and a framework region 4,
(i) an amino acid sequence wherein an amino acid sequence corresponding to not less than one particular region of the amino acid sequence shown by SEQ ID No: 4 has been exchanged for an amino acid sequence corresponding to the same kind of not less than one particular region contained in an amino acid sequence shown by SEQ ID No: 8, an amino acid sequence shown by SEQ ID No: 12, an amino acid sequence shown by SEQ ID No: 16 or an amino acid sequence shown by SEQ ID No: 20,
(ii) an amino acid sequence wherein an amino acid sequence corresponding to not less than one particular region of the amino acid sequence shown by SEQ ID No: 8 has been exchanged for an amino acid sequence corresponding to the same kind of not less than one particular region contained in an amino acid sequence shown by SEQ ID No: 4, an amino acid sequence shown by SEQ ID No: 12, an amino acid sequence shown by SEQ ID No: 16 or an amino acid sequence shown by SEQ ID No: 20,
(iii) an amino acid sequence wherein an amino acid sequence corresponding to not less than one particular region of the amino acid sequence shown by SEQ ID No: 12 has been exchanged for an amino acid sequence corresponding to the same kind of not less than one particular region contained in an amino acid sequence shown by SEQ ID No: 4, an amino acid sequence shown by SEQ ID No: 8, an amino acid sequence shown by SEQ ID No: 16 or an amino acid sequence shown by SEQ ID No: 20,
(iv) an amino acid sequence wherein an amino acid sequence corresponding to not less than one particular region of the amino acid sequence shown by SEQ ID No: 16 has been exchanged for an amino acid sequence corresponding to the same kind of not less than one particular region contained in an amino acid sequence shown by SEQ ID No: 4, an amino acid sequence shown by SEQ ID No: 8, an amino acid sequence shown by SEQ ID No: 12 or an amino acid sequence shown by SEQ ID No: 20, and
(v) an amino acid sequence wherein an amino acid sequence corresponding to not less than one particular region of the amino acid sequence shown by SEQ ID No: 20 has been exchanged for an amino acid sequence corresponding to the same kind of not less than one particular region contained in an amino acid sequence shown by SEQ ID No: 4, an amino acid sequence shown by SEQ ID No: 8, an amino acid sequence shown by SEQ ID No: 12 or an amino acid sequence shown by SEQ ID No: 16;
[a²] a protein having an amino acid sequence shown by SEQ ID No: 2, an amino acid sequence shown by SEQ ID No: 6, an amino acid sequence shown by SEQ ID No: 10, an amino acid sequence shown by SEQ ID No: 14, an amino acid sequence shown by SEQ ID No: 18 or an amino acid sequence of the protein of [a¹], wherein one or more amino acids have been deleted, substituted or added, which binds to a female sex hormone after forming a complex with a protein having an amino acid sequence shown by SEQ ID No: 4, an amino acid sequence shown by SEQ ID No: 8, an amino acid sequence shown by SEQ ID No: 12, an amino acid sequence shown by SEQ ID No: 16; an amino acid sequence shown by SEQ ID No: 20 or an amino acid sequence of the protein of [b¹];
[b²] a protein having an amino acid sequence shown by SEQ ID No: 4, an amino acid sequence shown by SEQ ID No: 8, an amino acid sequence shown by SEQ ID No: 12, an amino acid sequence shown by SEQ ID No: 16; an amino acid sequence shown by SEQ ID No: 20 or an amino acid sequence of the protein of [b¹], wherein one or more amino acids have been deleted, substituted or added, which binds to a female sex hormone after forming a complex with a protein having an amino acid sequence shown by SEQ ID No: 2, an amino acid sequence shown by SEQ ID No: 6, an amino acid sequence shown by SEQ ID No: 10, an amino acid sequence shown by SEQ ID No: 14, an amino acid sequence shown by SEQ ID No: 18 or an amino acid sequence of the protein of [a¹];
[a³] a protein having an amino acid sequence shown by SEQ ID No: 2, an amino acid sequence shown by SEQ ID No: 6, an amino acid sequence shown by SEQ ID No: 10, an amino acid sequence shown by SEQ ID No: 14, an amino acid sequence shown by SEQ ID No: 18 or an amino acid sequence of the protein of [a¹], wherein one or more amino acids have been deleted, substituted or added, which binds to a female sex hormone after forming a complex with a protein having an amino acid sequence shown by SEQ ID No: 4, an amino acid sequence shown by SEQ ID No: 8, an amino acid sequence shown by SEQ ID No: 12, an amino acid sequence shown by SEQ ID No: 16, SEQ ID; an amino acid sequence shown by SEQ ID No: 20 or an amino acid sequence of the protein of [b¹], wherein one or more amino acids have been deleted, substituted or added;
[b³] a protein having an amino acid sequence shown by SEQ ID No: 4, an amino acid sequence shown by SEQ ID No: 8, an amino acid sequence shown by SEQ ID No: 12, an amino acid sequence shown by SEQ ID No: 16, SEQ ID; an amino acid sequence shown by SEQ ID No: 20 or an amino acid sequence of the protein of [b¹], wherein one or more amino acids have been deleted, substituted or added, which binds to a female sex hormone after forming a complex with a protein having an amino acid sequence shown by SEQ ID No: 2, an amino acid sequence shown by SEQ ID No: 6, an amino acid sequence shown by SEQ ID No: 10, an amino acid sequence shown by SEQ ID No: 14, an amino acid sequence shown by SEQ ID No: 18 or an amino acid sequence of the protein of [a¹], wherein one or more amino acids have been deleted, substituted or added.

3. The protein of claim 1 or 2, or a salt thereof, wherein the female sex hormone is estrogen or a synthetic female sex hormone.

4. The protein of claim 3, or a salt thereof, wherein the estrogen is estradiol, estrone or estriol.

5. The protein of claim 3, or a salt thereof, wherein the synthetic female sex hormone is ethynyl estradiol or diethylbestrol.

6. A method of genetically recombining a protein of any of claims 1 - 5.

7. A protein obtained by the method of claim 6, or a salt thereof.

8. A partial peptide of a protein of any of claims 1 - 5 and 7, or a salt thereof.

9. A polynucleotide encoding a protein or partial peptide of a protein of any of claims 1 - 5 and 7.

10. A recombinant vector containing a polynucleotide of claim 9.

11. A transformant transformed with the recombinant vector of claim 10.

12. A method of producing a protein of any of claims 1 - 5 and 7, or a partial peptide thereof, or a salt thereof, which comprises producing a protein of any of claims 1 - 5 and 7, or a partial peptide thereof, or a salt thereof, and harvesting the same.

13. A complex wherein a protein of the following [a] and a protein of the following [b] are linked:
[a] a protein having an amino acid sequence shown by SEQ ID No: 2, an amino acid sequence shown by SEQ ID No: 6, an amino acid sequence shown by SEQ ID No: 10, an amino acid sequence shown by SEQ ID No: 14, an amino acid sequence shown by SEQ ID No: 18 or an amino acid sequence substantially the same as these;
[b] a protein having an amino acid sequence shown by SEQ ID No: 4, an amino acid sequence shown by SEQ ID No: 8, an amino acid sequence shown by SEQ ID No: 12, an amino acid sequence shown by SEQ ID No: 16, SEQ ID; an amino acid sequence shown by SEQ ID No: 20 or an amino acid sequence substantially the same as these.

14. A complex of claim 13, which is a single chain antibody.

15. A phage that expresses the single chain antibody of claim 14.

16. A method of identifying a female sex hormone binding to a complex of claim 13 or 14, which comprises using said complex or the phage of claim 15.

17. A method of measuring or quantifying a female sex hormone, which comprises using the complex of claim 13 or 14 or the phage of claim 15.

18. A kit for measuring or quantifying a female sex hormone, which comprises the complex of claim 13 or 14 or the phage of claim 15.

19. A method of concentrating a female sex hormone, which comprises using the complex of claim 13 or 14 or the phage of claim 15.

20. A kit for concentrating a female sex hormone, which comprises the complex of claim 13 or 14 or the phage of claim 15.
